# EUROPEAN PATENT APPLICATION

(11) **EP 2 455 403 A1**
(43) Date of publication of application: **23.05.2012**
(21) Application number: 10306286.5
(22) Date of filing: 23.11.2010
(51) Int. Cl.: C07K 16/28, A61K 39/395, A61P 35/00

(54) **Homogeneous humanized antibodies against JAM-A that inhibit proliferation**

(71) Applicant: PIERRE FABRE MEDICAMENT, 92100 Boulogne-Billancourt (FR)
(72) Inventor: Goetsch, Liliane, 74130 Ayze (FR); Corvaia, Nathalie, 74160 Collonges sous Saleve (FR); Haeuw, Jean-François, 74160 Beaumont (FR); Bes, Cédric, 30250 Villevieille (FR)
(74) Representative: Warcoin, Jacques

(57) **Abstract**

The present invention relates to humanized antibodies able to inhibit tumor growth, as well as the amino and nucleic acid sequences coding for such antibodies. From one aspect, the invention relates to anti-JAM-A homogeneous humanized antibodies able to inhibit tumor growth. The invention also comprises the use of such antibodies as a drug for the preventive and/or therapeutic treatment of cancers, as well as compositions comprising such antibodies in combination with other anticancer compounds. The invention also relates to a method for the preparation of such humanized antibodies.

## Description

The present invention relates to novel humanized antibodies able to inhibit tumor growth, as well as the amino and nucleic acid sequences coding for such antibodies. From one aspect, the invention relates to novel anti-JAM-A homogeneous humanized antibodies able to inhibit tumor growth. The invention also comprises the use of such antibodies as a drug for the preventive and/or therapeutic treatment of cancers, as well as compositions comprising such antibodies in combination with other anticancer compounds. The invention also relates to a method for the preparation of such novel humanized antibodies.

One of the most promising therapeutics against human cancer and other diseases is that of antibody therapy.

The terms "antibody", "antibodies" or "immunoglobulin" are used interchangeably in the broadest sense and include monoclonal antibodies (e.g., full length or intact monoclonal antibodies), polyclonal antibodies, multivalent antibodies or multispecific antibodies (e.g., bispecific antibodies so long as they exhibit the desired biological activity).

Typically, such molecule consists of a glycoprotein comprising at least two heavy (H) chains and two light (L) chains inter-connected by disulfide bonds. Each heavy chain is comprised of a heavy chain variable region (or domain) (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions(FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immunue system (e.g. effector cells) and the first component (Clq) of the classical complement system.

They may also include certain antibody fragments, as described in greater detail herein, thereof which exhibit the desired binding specificity and affinity, regardless of the source or immunoglobulin type (i.e., IgG, IgE, IgM, IgA, etc.).

In general, for the preparation of monoclonal antibodies or their functional fragments, especially of murine origin, it is possible to refer to techniques which are described in particular in the manual "Antibodies" (Harlow and Lane, Antibodies: A Laboratory Manual, Cold Spring Harbor Laboratory, Cold Spring Harbor NY, pp. 726, 1988) or to the technique of preparation from hybridomas described by Kohler and Milstein (Nature, 256:495-497, 1975).

Typically, antibodies are derived from non-human sources, whose application in humans necessitates reduction of their immunogenicity when administered to human subjects.

Mouse monoclonal antibodies are highly immunogenic in humans, thus limiting their potential use for cancer therapy, especially when repeated administration is necessary. Various methodologies have been developed to address this issue. First, to reduce the immunogenicity of mouse monoclonal antibodies, chimeric monoclonal antibodies were generated, with the variable Ig domains of a mouse monoclonal antibody being fused to human constant Ig domains.

Though some chimeric monoclonal antibodies have proved less immunogenic in humans, the mouse variable Ig domains can still lead to a significant human anti-mouse response.

Full humanization is feasible by introducing the six CDRs from the mouse heavy and light chain variable Ig domains into the appropriate framework regions of human variable Ig domains. This CDR grafting technique takes advantage of the conserve structure of the variable Ig domains, with the four framework regions (FR1-FR4) serving as a scaffold to support the CDR loops which are the primary contacts with antigen.

A drawback, however, of the CDR grafting technique is the fact that amino acids of the framework regions can contribute to antigen binding, as well as amino acids of the CDR loops can influence the association of the two variable Ig domains. To maintain the affinity of the humanized monoclonal antibody, the CDR grafting technique relies on the proper choice of the human framework regions and site-directed mutagenesis of single amino acids aided by computer modeling of the antigen binding site.

As described in the published patent application WO2008/062063, part of the prior art, the applicant has reported the generation by functional approaches of a murine anti-tumoral antibody, referred as 6F4 or m6F4, which binds to the protein JAM-A and is capable of inhibiting *in vitro* and/or *in vivo*, in a significant manner, the proliferation of tumor cells.

JAM-A is a membrane protein belonging to the immunoglobulin superfamily (IgSF), in which it belongs to the junctional adhesion molecule (JAM) family. In man, the JAM family comprises several members, including the JAM-A, JAM-B, JAM-C, A33 and A34 proteins. Among the members of the JAM family, JAM-A has the highest homology with JAM-B and JAM-C, approximately 35% sequence identity in amino acids and 45% similarity with these two proteins. JAM-A protein is also called JAM A, F11R, F11 receptor, JAM-1, JAM 1, PAM-1 or CD321.

Two isoforms, A and B respectively, of the JAM-A precursor differing by the length of the extracellular region were identified:

The protein expressed on the surface of the human cells has a single polypeptide chain with an intracellular C-terminal domain, a single transmembrane domain (21 amino acids) and an N-terminal extracellular region containing two "Ig-like" domains.

JAM-A has an N-glycosylation site, an Asn residue in position 185 for isoform A and 145 for isoform B, and two disulfide bridges, one between Cys residues 50 and 109 in the Ig N-terminal domain and one between residues Cys 153 and 212 in the second Ig domain.

The presence of the two extracellular Ig-like domains was confirmed by crystallography (Kostrewa et al., 2001, EMBO J. 16:4391-4398; Prota et al., 2003, Proc. Natl. Acad. Sci. USA, 100:5366-5371). These two domains are connected by a tripeptide linker (sequence VLV [127-129], isoform A). These structural studies also confirmed the implication of JAM-A in homophilic interactions on the cell surface involving the extracellular region; this region, produced in recombinant form and capable of forming homodimers in solution (Bazzoni et al., 2000, J. Biol. Chem. 275:30970-30976) also made it possible to identify the amino acids involved in these interactions: Arg 59, Glu 61, Lys 63, Leu 72, Tyr 75, Met 110, Glu 114, Tyr 119 and Glu 121. The tripeptide RVE [59-61] is relatively conserved within the JAM family (RLE for JAM-B, RIE for JAM-C) and constitutes the minimal motif for homodimer formation (Kostrewa et al., 2001, EMBO J. 16:4391-4398).

In epithelial and endothelial cells, JAM-A is mainly found in the tight junctions (Liu et al., 2000, J. Cell Sci., 113:2363-2374). The cytoplasmic region contains a type II PDZ domain in the C-terminal position (sequence FLV [298-300], isoform a, which is responsible for the interaction of JAM-A with various cytosolic proteins associated with the tight junction, also containing a PDZ domain, such as ZO-1, AF-6, MUPP-1 and PAR-3 (Ebnet et al., 2000, J. Biol. Chem., 275:27979-27988; Itoh et al., 2001, J. Cell Biol., 154:491-498; Hamazaki et al., 2002, J. Biol. Chem., 277:455-461). Murine antibodies directed against the region [111-123] involved in dimer formation, so-called J3F.1 and J10.4 antibodies, are capable of inhibiting the homodimerization of JAM-A and the reconstruction of the epithelial barrier *in vitro* (Mandell et al., 2004, J. Biol. Chem., 279:16254-16262).

JAM-A interacts with integrin αᵥβ₃ and is involved in the migration of endothelial cells on vitronectin, ligand of integrin αᵥβ₃ (Naik and Naik, 2005, J. Cell Sci. 119:490-499). Anti-JAM-A antibody J3F.1, in the same manner as an anti-αᵥβ₃ antibody, inhibits the migration of endothelial cells and the angiogenesis induced by bFGF *in vitro* (Naik et al., 2003, Blood, 102:2108-2114). Various signaling pathways were demonstrated in endothelial cells: MAP kinases, PI3-kinase and PKC (Naik et Naik, 2005, J. Cell Sci., 119:490-499; Naik et al., 2003, Blood, 102:2108-2114; Naik et al., 2003, Artherioscler. Thromb. Vasc. Biol., 23:2165-2171).

JAM-A is also expressed in monocytes, lymphocytes, neutrophils and platelets (Williams et al., 1999, Mol. Immunol., 36:1175-1188). JAM-A protein was however initially identified as a receptor of the F11 antibody, an antibody capable of activating platelets and inducing their aggregation (Naik et al., 1995, Biochem. J., 310:155-162; Sobocka et al., 2000, Blood, 95:2600-2609). Peptides [28-60] and [97-109] belong to the F11 antibody epitope and are involved in platelet activation and aggregation phenomena and in homodimerization (Babinska et al., 2002, Thromb. Haemost., 87:712-721).

Rat antibody BV11, directed against the murine form of JAM-A, inhibits the trans-endothelial migration of monocytes *in vitro* and *in vivo* (Del Maschio et al., 1999, J. Exp. Med., 190:1351-1356). Ostermann and colleagues (2002, Nature Immunol., 3:151-158) showed that JAM-A was a ligand of α_{L}β₂ or LFA-1 (lymphocyte function-associated antigen 1) integrin, which is overexpressed in response to certain chemokines during the development of an anti-inflammatory response and is required for the diapedesis or migration of leukocytes to the site of inflammation. JAM-A, via the second Ig-like domain, contributes to the adhesion and trans-endothelial migration of T lymphocytes and neutrophils (Ostermann et al., 2002, Nature Immunol., 3:151-158), and thus plays an important role in the recruitment of leukocytes to the site of inflammation.

JAM-A protein is also implicated in viral infection phenomena. JAM-A is indeed a receptor of reovirus, viruses responsible for certain types of encephalitis by means of interacting with attachment protein σ1 (Barton et al., 2001, Cell 104:441-451). Anti-JAM-A antibody J10.4 inhibits the binding of reovirus to JAM-A (Forrest et al., 2003, J. Biol. Chem., 278:48434-48444).

Despite the patent application WO2008/062063 has been published, and must be considered as part of the prior art, the 6F4 antibody can be described as an isolated murine antibody, or a derived compound or functional fragment of same, capable of inhibiting the proliferation of tumor cells *in vitro* and/or *in vivo*; said antibody, or a derived compound or functional fragment of same, comprising i) a light chain comprising, according to IMGT, the CDR-L1 of the sequence SEQ ID No. 1 or of a sequence with at least 80% identity after optimal alignment with sequence SEQ ID No. 1 ; the CDR-L2 of the sequence SEQ ID No. 2 or of a sequence with at least 80% identity after optimal alignment with sequence SEQ ID No. 2; and the CDR-L3 of the sequence SEQ ID No. 3 or of a sequence with at least 80% identity after optimal alignment with sequence SEQ ID No. 3, and ii) a heavy chain comprising, according to IMGT, the CDR-H1 of the sequence SEQ ID No. 4 or of a sequence with at least 80% identity after optimal alignment with sequence SEQ ID No. 4; the CDR-H2 of the sequence SEQ ID No. 5 or of a sequence with at least 80% identity after optimal alignment with sequence SEQ ID No. 5 and the CDR-H3 of the sequence SEQ ID No. 6 or of a sequence with at least 80% identity after optimal alignment with sequence SEQ ID No. 6.

The IMGT unique numbering has been defined to compare the variable domains whatever the antigen receptor, the chain type, or the species [Lefranc M.-P., Immunology Today 18, 509 (1997) / Lefranc M.-P., The Immunologist, 7, 132-136 (1999) / Lefranc, M.-P., Pommié, C., Ruiz, M., Giudicelli, V., Foulquier, E., Truong, L., Thouvenin-Contet, V. and Lefranc, Dev. Comp. Immunol., 27, 55-77 (2003)]. In the IMGT unique numbering, the conserved amino acids always have the same position, for instance cystein 23 (1st-CYS), tryptophan 41 (CONSERVED-TRP), hydrophobic amino acid 89, cystein 104 (2nd-CYS), phenylalanine or tryptophan 118 (J-PHE or J-TRP). The IMGT unique numbering provides a standardized delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117. As gaps represent unoccupied positions, the CDR-IMGT lengths (shown between brackets and separated by dots, e.g. [8.8.13]) become crucial information. The IMGT unique numbering is used in 2D graphical representations, designated as IMGT Colliers de Perles [Ruiz, M. and Lefranc, M.-P., Immunogenetics, 53, 857-883 (2002) / Kaas, Q. and Lefranc, M.-P., Current Bioinformatics, 2, 21-30 (2007)], and in 3D structures in IMGT/3Dstructure-DB [Kaas, Q., Ruiz, M. and Lefranc, M.-P., T cell receptor and MHC structural data. Nucl. Acids. Res., 32, D208-D210 (2004)].

By amino acid sequence having at least 80%, preferably 85%, 90%, 95% and 98% identity with a reference amino acid sequence, those having, with respect to the reference sequence, certain modifications, in particular a deletion, addition or substitution of at least one amino acid, a truncation or an elongation are preferred. In the case of a substitution of one or more consecutive or nonconsecutive amino acid(s), the substitutions are preferred in which the substituted amino acids are replaced by "equivalent" amino acids. The expression "equivalent amino acids" is aimed here at indicating any amino acid capable of being substituted with one of the amino acids of the base structure without, however, essentially modifying the biological activities of the corresponding antibodies and such as will be defined later, especially in the examples. These equivalent amino acids can be determined either by relying on their structural homology with the amino acids which they replace, or on results of comparative trials of biological activity between the different antibodies capable of being carried out.

By "antigen binding fragment" or "functional fragment" of an antibody, it is intended to indicate in particular an antibody fragment, such as Fv, scFv (sc for single chain), Fab, F(ab')₂, Fab', scFv-Fc fragments or diabodies, or any fragment of which the half-life time would have been increased by chemical modification, such as the addition of poly(alkylene) glycol such as poly(ethylene) glycol ("PEGylation") (pegylated fragments called Fv-PEG, scFv-PEG, Fab-PEG, F(ab')₂-PEG or Fab'-PEG) ("PEG" for Poly(Ethylene) Glycol), or by incorporation in a liposome, said fragments having at least one of the characteristic CDRs of the antibody according to the invention, and, especially, in that it is capable of exerting in a general manner an even partial activity of the antibody from which it is descended, such as in particular the capacity to recognize and to bind to JAM-A.

A "derivative" or "derived compound" of an antibody means in particular a binding protein composed of a peptide scaffold and at least one of the CDRs of the original antibody in order to preserve its ability to be recognized. Such derived compounds, well-known to a person skilled in the art, will be described in more detail later in the present description.

It must be understood here that the invention does not relate to antibodies in natural form, i.e., they are not taken from their natural environment but are isolated or obtained by purification from natural sources or obtained by genetic recombination or chemical synthesis and thus they can carry unnatural amino acids as will be described below.

The murine antibody 6F4 can also be described as an antibody comprising a light-chain of amino acid sequence SEQ ID No. 7 and a heavy-chain of amino acid sequence SEQ ID No. 8.

This monoclonal antibody 6F4, or its derived compounds or functional fragments, is secreted by the hybridoma filed with the CNCM on July 6, 2006, under number I-3646.

By classical CDR grafting, as described above, the applicant has generated a humanized version of the murine 6F4 Mab. This first humanized version, referred hereinafter as Hz6F4-1 (or Hz6F4 1 or 6F4Hz-1 or also 6F4HZ1), has been classically obtained by introduction of the 6 CDRs of the murine 6F4 into human germlines. This humanization process was described in the patent application W02008/062063.

Surprisingly, the Hz6F4-1 does not present common drawbacks for humanized antibody as above described (loss of affinity or modification in the association of the two variable Ig domains) but presents particular drawbacks in relation with its developability and manufacturabilty.

More particularly, as it will be illustrated hereinafter in the examples, the purified Hz6F4-1 has a very unusual heterogeneous profile, including numerous charge variants, being an omen of future difficulties in the course of the pharmaceutical development and the manufacture of said antibody.

Another major drawback of the Hz6F4-1 is linked to its acidic isoelectric point that will not allow efficient virus inactivation, which is a mandatory step for an antibody produced in a mammalian cell line.

Generally speaking, an acidic isoelectric point for an IgG type monoclonal antibody can be considered as an isoelectric point under 7.

Generic purification platforms follow a traditional scheme with three major chromatography steps. Protein A chromatography is used for the initial capture step. The eluate is then held at low pH to inactivate virus and then adjusted to increase the pH before the cation exchange chromatography step (CEX). After cation exchange a diafiltration step is required to prepare the product for the polishing step witch is carried out using anionic exchangers (AEX) in a flow through mode. The anion exchange step can be base on chromatography media or membrane absorbers. In the great majority of cases this platform is efficient and robust because generally mAbs have high isoelectric points (>7).

Some problems issued from the purification of Hz6F4-1 which has an isoelectric point below 7, close to 5.8. Low pH virus inactivation was not really possible with Hz6F4-1 because it was not stable when pH is lower than 4.

To keep an acceptable binding capacity on cation exchange chromatography media, the load pH had to be lower than 4, which was an issue because of Hz6F4-1 instability at this pH value. On the other hand, the more the CEX load pH was low, the more the level of impurities such as host cell proteins (HCP), virus, DNA and endotoxins that were retained with the mAb on the chromatography media was high. Process HCP clearance was reduced because most of the Chinese Hamster Ovary cells Proteins (CHOP) have an isoelectric point closer to that of Hz6F4-1. Virus removal by the anion exchange step in a flow through mode would likely have been affected by the required load pH.

It is the first time such technical problems are reported in the field of the humanization of antibody.

The present invention relies on the observations made by the applicant and the modifications performed to solve this issue.

More particularly, the present invention relates to a method for the preparation of a novel variant humanized antibody of the murine 6F4, referred as Hz6F4-2 (or Hz6F4 2 or 6F4Hz-2 or also 6F4HZ2).

According a general aspect of the invention, it is described here, for the first time, a method for the preparation of a high-homogeneous variant of a parental humanized antibody, or a derivative or antigen binding fragment thereof, said parental humanized antibody comprising a light chain variable domain having the 3 CDRs sequences SEQ ID Nos. 1, 2 and 3 and a heavy chain variable domain having the 3 CDRs sequences SEQ ID Nos. 4, 5 and 6, and said humanized antibody being capable of binding to JAM-A, wherein said method comprises the step of:
a) the substitution with a neutral or basic amino acid residue of at least one amino acid residue of said parental humanized antibody light chain variable domain or of said parental humanized antibody heavy chain variable domain; and
b) the selection of the variant of said humanized antibody obtained in step a) as a high-homogeneous variant thereof whether said substitution results in the increase of the pI of the variant humanized antibody compared with the parental humanized antibody.

An antibody characterized as "high homogeneous", it must be understood, as it will be apparent in the following examples, means an antibody showing a homogeneous profile in terms of glycation, mass, etc., resulting in an improved developability, manufacturability and overall drugability profile. For example, in the present case, the high homogeneous variant of the invention contain less multiple charge variants like glycation residues and less higher mass variants compared to the parental antibody. This expression will be clearly obvious for the man skilled in the art regarding the following examples.

The isoelectric point (pI), sometimes abbreviated to IEP, is the pH at which the antibody carries a net electrical charge equal to zero. Theoretical pI can be calculated by software and then determined by analitycal methods such as IsoElectric Focusing (IEF) or Capillary IEF (cIEF).

Similarly, the strategy for the selection of amino acid substitution will also be apparent for the man skilled in the art at the reading of the following examples.

An amino acid substitution increasing the pI of the antibody can comprise the substitution of an acidic amino acid by a neutral or a basic amino acid as well as the substitution of a neutral amino acid by a basic amino acid.

Neutral amino acids can be selected in the group consisting of Alanine (A), Valine (V), Leucine (L), Isoleucine (I), Proline (P), Methionine (M), Phenylalanine (F), Tryptophan (W), Glycine (G), Serine (S), Threonine (T), Cystein (C), Asparagine (N), Glutamine (Q) or Tyrosine (Y).

Acidic amino acids can be selected in the group consisting of Aspartic Acid (D) or Glutamic acid (E).

Basic amino acids can be selected in the group consisting of Lysine (K), Arginine (R) or Histidine (H).

In a preferred embodiment, in the step a) of the method according to the invention, at least one amino acid residue selected from D1, Q3, Q24 and E55 of the light chain variable domain having the sequence SEQ ID No. 9 is substituted with a neutral or basic amino acid residue.

As a consequence, the invention also relates to a variant humanized antibody, or a derivative or antigen binding fragment thereof, characterized in that it comprises the light chain variable domain of a parental antibody of sequence SEQ ID No. 9 wherein at least one amino acid residue selected from D1, Q3, Q24 and E55 is substituted with an amino acid residue resulting in an increase of the positive charge of said light chain.

It must be understood here that the increase of the pI results in an increase of the net positive charge. As a consequence, a substitution increasing the pI should be considered as equivalent to a substitution increasing the positive charge.

In another preferred embodiment, in the step a) of the method according to the invention, at least one amino acid residue selected from E1, Q38, G44, E62 and D77 of the heavy chain variable domain having the sequence SEQ ID No. 10 is substituted with a neutral or basic amino acid residue.

As a consequence, the invention also relates to a variant humanized antibody, or a derivative or antigen binding fragment thereof, characterized in that it comprises the heavy chain variable domain of a parental antibody of sequence SEQ ID No. 10 wherein at least one amino acid residue selected from E1, Q38, G44, E62 and D77 is substituted with an amino acid residue resulting in an increase of the positive charge of said heavy chain.

In another aspect, the invention comprises a method for the preparation of a high-homogeneous variant of a parental humanized antibody according to the invention, wherein in step a):
- at least one amino acid residue selected from D1, Q3, Q24 and E55 of the light chain variable domain having the sequence SEQ ID No. 9 is substituted with a neutral or basic amino acid residue, and
- at least one amino acid residue selected from E1, Q38, G44, E62 and D77 of the heavy chain variable domain having the sequence SEQ ID No. 10 is substituted with a neutral or basic amino acid residue.

Another particular preferred aspect of the invention relates to a method for the preparation of a high-homogeneous variant of a parental humanized antibody as above described, wherein in step a):
i) at least one amino acid residue selected from D1, Q3, Q24 and E55 of the light chain variable domain having the sequence SEQ ID No. 9 is substituted with the amino acid residue A, R, K and Q, respectively; and
ii) at least one amino acid residue selected from E1, Q38, G44, E62 and D77 of the heavy chain variable domain having the sequence SEQ ID No. 10 is substituted with the amino acid residue Q, R, R, Q and S, respectively.

As a consequence, the invention also describes a variant humanized antibody, or a derivative or antigen binding fragment thereof, characterized in that it comprises:
i) the light chain variable domain of the parental antibody of sequence SEQ ID No. 9 wherein at least one amino acid residue selected from D1, Q3, Q24 and E55 is substituted with the amino acid residue A, R, K and Q, respectively ; and
ii) the heavy chain variable domain of the parental antibody of sequence SEQ ID No. 10 wherein at least one amino acid residue selected from E1, Q38, G44, E62 and D77 is substituted with the amino acid residue Q, R, R, Q and S, respectively.

More particularly, the preferred variant of the invention consists in the variant humanized antibody, or a derivative or antigen binding fragment thereof, which is characterized in that it comprises:
i) a light chain variable domain comprising the sequence SEQ ID N° 11, and
ii) a heavy chain variable domain comprising the sequence SEQ ID N° 12.

For more clarity, the following table 1 summarizes the amino acids sequences of the invention.

**Table 1**

| **Antibody 6F4** | **Heavy chain** | **Light chain** | **SEQ ID NO.** |
|---|---|---|---|
| CDR(s) | - | CDR-L1 | 1 |
| | - | CDR-L2 | 2 |
| | - | CDR-L3 | 3 |
| | CDR-H1 | - | 4 |
| | CDR-H2 | - | 5 |
| | CDR-H3 | - | 6 |
| murine | - | VL | 7 |
| | VH | | 8 |
| Parental humanized | - | VL | 9 |
| | VH | - | 10 |
| Humanized Hz-2 | - | VL | 11 |
| | VH | - | 12 |

For the man skilled in the art, any modification of an amino acid by an equivalent amino acid can be considered as obvious and, as a consequence, as part of the invention.

Here, the expression "equivalent amino acids" is meant to indicate any amino acids likely to be substituted for one of the structural amino acids without however modifying the biological activities of the corresponding antibodies and of those specific examples defined below and, more particularly, without modifying the pI of the corresponding antibodies. For example an acidic amino acid could be substituted by another equivalent acidic amino acid.

In another complementary preferred embodiment on the invention, the variant humanized antibody, or a derivative or antigen binding fragment thereof, is characterized in that its pI is comprised between 7 and 9, preferably between 7 and 8.

As it will be more apparent for the man skilled in the art in the examples, the increase of the pI of both the light and the heavy chains variable regions, has an important impact on the homogeneity of the Hz6F4-2.

This aspect of the invention is of a particular interest as such an approach has not only never been described but also is contrary to the teaching of the prior art.

Actually, the prior art teaches that lowering the pI could be an advantage for non-aggregation optimization (Arbabi-Ghahroudi & al., PEDS, Vol.22, no.2 pp59-66, 2009).

It is also known from the prior art to lower the pI, by engineering the variable regions for example, in order to reduce the elimination of IgG antibodies, in other word, to improve the half-life of antibodies (Igawa & al., PEDS, Vol.23, no.5 pp. 385-392, 2010).

The present invention clearly overcome a technical prejudice of the prior art as it is the first time it is suggested to increase the pI of an antibody.

According to still another aspect, the variant humanized antibody of the invention, or its derived compounds or functional fragments, is characterized in that it has a K_{D} for JAM-A between roughly 100 nM and roughly 100 pM. More preferably, said K_{D} for JAM-A is between roughly 50 nM and roughly 1 nM.

The expression "K_{D}" refers to the dissociation constant of a given antibody-antigen complex. K_{D}=K_{off}/Kₒₙ with K_{off} consisting of the "off rate" constant for the dissociation of the antibody from the antibody-antigen complex and Kₒₙ consisting of the level at which the antibody binds the antigen (Chen Y. et al., 1999, J.Mol.Biol., 293:865-881).

A novel aspect of the present invention relates to an isolated nucleic acid characterized in that it is selected among the following nucleic acids (including any degenerate genetic code):
a) a nucleic acid, DNA or RNA, coding for an antibody, or for a derivative or antigen binding fragment thereof, according to the invention;
b) a nucleic acid comprising the light chain of nucleic acid sequence SEQ ID No. 13 and the heavy chain of nucleic acid sequence SEQ ID No. 14, said SEQ ID No. 13 and SEQ ID No. 14 encoding respectively the light chain of the variant humanized antibody having the sequence SEQ ID No. 11 and the heavy chain of the variant humanized antibody having the sequence SEQ ID No. 12; and
c) a nucleic acid complementary to a nucleic acid as defined in a) or b).

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, defining a fragment or a region of a nucleic acid, containing unnatural nucleotides or not, and being either a double-strand DNA, a single-strand DNA or transcription products of said DNAs.

It should also be included here that the present invention does not relate to nucleotide sequences in their natural chromosomal environment, i.e., in a natural state. The sequences of the present invention have been isolated and/or purified, i.e., they were sampled directly or indirectly, for example by a copy, their environment having been at least partially modified. Isolated nucleic acids obtained by recombinant genetics, by means, for example, of host cells, or obtained by chemical synthesis should also be mentioned here.

"Nucleic sequences exhibiting a percentage identity of at least 80%, preferably 85%, 90%, 95% and 98%, after optimal alignment with a preferred sequence" means nucleic sequences exhibiting, with respect to the reference nucleic sequence, certain modifications such as, in particular, a deletion, a truncation, an extension, a chimeric fusion and/or a substitution, notably punctual. Preferably, these are sequences which code for the same amino acid sequences as the reference sequence, this being related to the degeneration of the genetic code, or complementarity sequences that are likely to hybridize specifically with the reference sequences, preferably under highly stringent conditions, notably those defined below.

Hybridization under highly stringent conditions means that conditions related to temperature and ionic strength are selected in such a way that they allow hybridization to be maintained between two complementarity DNA fragments. On a purely illustrative basis, the highly stringent conditions of the hybridization step for the purpose of defining the polynucleotide fragments described above are advantageously as follows.

DNA-DNA or DNA-RNA hybridization is carried out in two steps: (1) prehybridization at 42°C for three hours in phosphate buffer (20 mM, pH 7.5) containing 5X SSC (1X SSC corresponds to a solution of 0.15 M NaCl + 0.015 M sodium citrate), 50% formamide, 7% sodium dodecyl sulfate (SDS), 10X Denhardt's, 5% dextran sulfate and 1% salmon sperm DNA; (2) primary hybridization for 20 hours at a temperature depending on the length of the probe (i.e.: 42°C for a probe >100 nucleotides in length) followed by two 20-minute washings at 20°C in 2X SSC + 2% SDS, one 20-minute washing at 20°C in 0.1X SSC + 0.1% SDS. The last washing is carried out in 0.1X SSC + 0.1% SDS for 30 minutes at 60°C for a probe >100 nucleotides in length. The highly stringent hybridization conditions described above for a polynucleotide of defined size can be adapted by a person skilled in the art for longer or shorter oligonucleotides, according to the procedures described in Sambrook, et al. (Molecular cloning: a laboratory manual, Cold Spring Harbor Laboratory; 3rd edition, 2001).

The invention also relates to a vector comprising a nucleic acid as described in the invention.

The invention notably targets cloning and/or expression vectors that contain such a nucleotide sequence.

The vectors of the invention preferably contain elements which allow the expression and/or the secretion of nucleotide sequences in a given host cell. The vector thus must contain a promoter, translation initiation and termination signals, as well as suitable transcription regulation regions. It must be able to be maintained in a stable manner in the host cell and may optionally have specific signals which specify secretion of the translated protein. These various elements are selected and optimized by a person skilled in the art according to the host cell used. For this purpose, the nucleotide sequences can be inserted in self-replicating vectors within the chosen host or be integrative vectors of the chosen host.

Such vectors are prepared by methods typically used by a person skilled in the art and the resulting clones can be introduced into a suitable host by standard methods such as lipofection, electroporation, heat shock or chemical methods.

The vectors are, for example, vectors of plasmid or viral origin. They are used to transform host cells in order to clone or express the nucleotide sequences of the invention.

The invention also comprises host cells transformed by or comprising a vector as described in the present invention.

The host cell can be selected among prokaryotic or eukaryotic systems such as bacterial cells, for example, but also yeast cells or animal cells, notably mammal cells. Insect or plant cells can also be used.

The invention also relates to animals, other than man, that have a transformed cell according to the invention.

Another aspect of the invention relates to a method for the production of a variant humanized antibody according to the invention, or derivative or antigen binding fragment thereof, characterized in that said method comprises the following steps:
a) the culture in a medium of and the suitable culture conditions for a host cell according to the invention; and
b) the recovery of said variant humanized antibody, or derivative or antigen binding fragment, thus produced from the culture medium or from said cultured cells.

The transformed cells according to the invention are of use in methods for the preparation of recombinant polypeptides according to the invention. Methods for the preparation of polypeptide according to the invention in recombinant form, characterized in that said methods use a vector and/or a cell transformed by a vector according to the invention, are also comprised in the present invention. Preferably, a cell transformed by a vector according to the invention is cultured under conditions that allow the expression of the aforesaid polypeptide and recovery of said recombinant peptide.

As already mentioned, the host cell can be selected among prokaryotic or eukaryotic systems. In particular, it is possible to identify the nucleotide sequences of the invention that facilitate secretion in such a prokaryotic or eukaryotic system. An vector according to the invention carrying such a sequence can thus be used advantageously for the production of recombinant proteins to be secreted. Indeed, the purification of these recombinant proteins of interest will be facilitated by the fact that they are present in the supernatant of the cellular culture rather than inside host cells.

The polypeptides of the invention can also be prepared by chemical synthesis. One such method of preparation is also an object of the invention. A person skilled in the art knows methods for chemical synthesis, such as solid-phase techniques (see notably Steward et al., 1984, Solid phase peptides synthesis, Pierce Chem. Company, Rockford, 111, 2nd ed.) or partial solid-phase techniques, by condensation of fragments or by conventional synthesis in solution. Polypeptides obtained by chemical synthesis and capable of containing corresponding unnatural amino acids are also comprised in the invention.

The variant humanized antibodies, or the derivatives or antigen binding fragments of same, likely to be obtained by the method of the invention are also comprised in the present invention.

In a preferred embodiment of the invention, the bispecific antibody is a bivalent or tetravalent antibody.

Lastly, the present invention relates to the variant humanized antibody described above, or a derivative or antigen binding fragment thereof, for use as a drug.

The invention also relates to a composition comprising as an active ingredient a compound consisting of a variant humanized antibody of the invention, or a derivative or antigen binding fragment thereof. Preferably, said variant humanized antibody is supplemented by an excipient and/or a pharmaceutically acceptable carrier.

According to still another embodiment, the present invention also relates to a pharmaceutical composition as described above that comprises at least a second antitumor compound selected among the compounds capable of specifically inhibiting the tyrosine kinase activity of receptors such as IGF-IR, EGFR, HER2/neu, cMET, VEGFR or VEGF, or any other antitumor compound known to a person skilled in the art. In a second preferred aspect of the invention, said second compound can be selected among the antibodies anti-EGFR, anti-IGF-IR, antiHER2/neu, anti-cMET, VEGFR, VEGF, etc., isolated, or their functional fragments and derived compounds, capable of inhibiting the proliferative and/or anti-apoptotic and/or angiogenic and/or inductive activity of metastatic dissemination promoted by said receptors.

According to still another embodiment of the invention, the composition comprises, in addition, as a combination product for use in a simultaneous, separated or extended fashion, at least one inhibiter of the tyrosine kinase activity of receptors such as IGF-IR, EGFR, HER2/neu, cMET and VEGFR.

In another preferred embodiment, said inhibiter of the tyrosine kinase activity of these receptors is selected from the group comprising derived natural agents, dianilinophthalimides, pyrazolo- or pyrrolo-pyridopyrimidines or quinazolines. Such inhibiting agents, well-known to a person skilled in the art, are described in the literature (Ciardiello F., Drugs 2000, Suppl. 1, 25-32).

Another embodiment complementary to the invention consists of a composition as described above comprised of, in addition, as a combination product for simultaneous, separated or extended use, a cytotoxic/cytostatic agent.

"Simultaneous use" means the administration of both compounds of the composition comprised in a single dosage form.

"Separated use" means administration, at the same time, of both compounds of the composition, comprised in distinct dosage forms.

"Extended use" means the successive administration of both compounds of the composition, each comprised in a distinct dosage form.

Generally, the composition according to the invention considerably increases cancer treatment effectiveness. In other words, the therapeutic effect of the antibody of the invention is enhanced in an unexpected way by the administration of a cytotoxic agent. Another major subsequent advantage produced by a composition of the invention relates to the possibility of using lower effective doses of the active ingredient, thus making it possible to avoid or reduce the risks of the appearance of side effects, in particular the effect of the cytotoxic agent. Moreover, this composition makes it possible to achieve the expected therapeutic effect more quickly.

"Therapeutic anticancer agent" or "cytotoxic agent" means a substance which, when it is administered to a patient, treats or prevents the development of cancer in the patient. Non-limiting examples of such agents include "alkylating" agents, antimetabolites, antitumor antibiotics, mitotic inhibitors, inhibitors of chromatin functioning, antiangiogenics, antiestrogens, antiandrogens and immunomodulators.

Such agents, for example, are cited in VIDAL, on the page devoted to compounds related to oncology and hematology under the heading "Cytotoxic"; the cytotoxic compounds cited by reference to this document are cited herein as preferred cytotoxic agents.

In a particularly preferred embodiment, said composition of the invention as a combination product is characterized in that said cytotoxic agent is bound chemically to said variant humanized antibody for use simultaneously.

In a particularly preferred embodiment, said composition is characterized in that said cytotoxic/cytostatic agent is selected among the spindle inhibitors or stabilizers, preferably vinorelbine and/or vinflunine and/or vincristine.

In order to facilitate binding between said cytotoxic agent and the variant humanized antibody according to the invention, spacer molecules can be introduced between the two compounds to bind, such as the poly(alkylene)glycol polyethyleneglycol or the amino acids; or, in another embodiment, said cytotoxic agents' active derivatives, into which have been introduced functions capable of reacting with said antibody, can be used. These binding techniques are well-known to a person skilled in the art and will not be discussed in more detail in the present description.

Another aspect of the invention relates to a composition characterized in that at least one of said antibodies, or of the derived compounds or functional fragments of same, is conjugated with a cellular toxin and/or a radioisotope.

Preferably, said toxin or said radioisotope is capable of preventing the growth or proliferation of the tumor cell, notably of completely inactivating said tumor cell.

Also preferably, said toxin is an enterobacteria toxin, notably *Pseudomonas* exotoxin A.

The radioisotopes preferentially combined with therapeutic antibodies are radioisotopes that emit gamma rays, preferentially iodine¹³¹, yttrium⁹⁰, gold¹⁹⁹, palladium¹⁰⁰, copper⁶⁷, bismuth²¹⁷ and antimony²¹¹. Radioisotopes that emit alpha and beta rays can also be used in therapy.

"Toxin or radioisotope combined with at least one antibody of the invention, or a functional fragment of same" refers to any means that makes it possible to bind said toxin or said radioisotope to that at least one antibody, notably by covalent binding between the two compounds, with or without the introduction of the binding molecule.

Examples of agents that allow chemical (covalent), electrostatic, or non-covalent bonding of all or part of the conjugate's elements include, in particular, benzoquinone, carbodiimide and more particularly EDC (1-ethyl-3-[3-dimethyl-aminopropyl]-carbodiimide-hydrochloride), dimaleimide, dithiobis-nitrobenzoic (DTNB) acid, N-succinimidyl S-acetyl thio-acetate (SATA), bridging agents with one or more groups, with one or more phenylaside groups, reacting with ultraviolet (UV) rays, most preferentially N-[-4 (azidosalicylamino)butyl]-3'-(2'-pyridyldithio)-propionamide (APDP), N-succinimid-yl 3(2-pyridyldithio) propionate (SPDP) and 6-hydrazino-nicotinamide (HYNIC).

Another form of binding, notably for radioisotopes, can consist of the use of bifunctional ion chelating agents.

Examples of such chelators include the chelators derived from EDTA (ethylenediaminetetraacetic acid) or DTPA (diethylenetriaminepentaacetic acid) which were developed to bind metals, particularly radioactive metals, with immunoglobulins. Thus, DTPA and its derivatives can be substituted on the carbon chain by various groups in such a way as to increase the stability and the rigidity of the ligand-metal complex (Krejcarek *et al*., 1977; Brechbiel *et al*., 1991; Gansow, 1991; US patent 4,831,175).

For example, DTPA (diethylenetriaminepentaacetic acid) and its derivatives, which long have been widely used in drug and biology either in its free form or in a complex with a metal ion, exhibit the remarkable characteristic of forming stable chelates with metal ions which can be coupled with proteins of therapeutic or diagnostic interest, such as antibodies, for the development of radio-immuno conjugates for cancer therapy (Meases *et al*., 1984; Gansow *et al*., 1990).

Also preferably, said at least one antibody of the invention forming said conjugate is selected among its functional fragments, notably fragments that have lost their Fc component, such as scFv fragments.

The present invention also comprises the use of the composition for the preparation of a drug, preferably intended for the prevention or the treatment of cancer.

The present invention also relates to the use of a variant humanized antibody, or a derivative or antigen binding fragment thereof, and/or of a composition according to the invention for the preparation of a drug for inhibiting the growth of tumor cells. Generally, the present invention relates to the use of a variant humanized antibody, or a derivative or antigen binding fragment thereof, and/or of a composition, for the preparation of a drug for cancer prevention or treatment.

The present invention also relates to the use of a variant humanized antibody, or a derivative or antigen binding fragment thereof, and/or of a composition according to the invention for the preparation of a drug for inhibiting the growth of tumor cells. Generally, the present invention relates to the use of a variant humanized antibody, or a derivative or antigen binding fragment thereof, and/or of a composition, for the preparation of a drug for the prevention or the treatment of a disease related to tumor cell proliferation.

Preferred cancers that can be prevented and/or treated include prostate cancer, osteosarcoma, lung cancer, breast cancer, endometrial cancer, colon cancer, multiple myeloma, ovarian cancer, pancreatic cancer or any other cancer.

In a preferred manner, said cancer is a cancer chosen among estrogen-related breast cancer, non-small cell lung cancer, colon cancer and/or pancreatic cancer.

Another aspect of the present invention relates to the use of the variant humanized antibody as described in a diagnostic method, preferably *in vitro*, of diseases related to JAM-A expression level. Preferably, said JAM-A protein related diseases in said diagnostic method will be cancers.

Thus, the variant humanized antibody of the invention, or any derivative or antigen binding fragment, can be employed in a method for the detection and/or quantification of JAM-A protein in a biological sample *in vitro*, notably for the diagnosis of diseases associated with an abnormal expression with this protein, such as cancers, wherein said method comprises the following steps:
a) placing the biological sample in contact with a variant humanized antibody according to the invention, or a derivative or antigen binding fragment thereof;
b) demonstrating the antigen-antibody complex possibly formed.

Thus, the present invention also comprises the kits or accessories for the implementation of a method as described, comprising the following elements:
a) a variant humanized antibody of the invention;
b) optionally, reagents for constituting the medium favorable to immunological reactions;
c) optionally, reagents that reveal the antigen-antibodies complexes produced by the immunological reaction.

Advantageously, the variant humanized antibody, or antigen binging fragments of same, can be immobilized on a support, notably a protein chip. One such protein chip is an object of the invention.

Advantageously, the protein chips can be used in the kits or accessories required for detecting and/or quantifying JAM-A protein in a biological sample.

It must be stated that the term "biological sample" relates herein to samples taken from a living organism (notably blood, tissue, organ or other samples taken from a mammal, notably man) or any sample likely to contain one such JAM-A protein (such as a sample of cells, transformed if needed).

Said variant humanized antibody can be in the form of an immunoconjugate or of a labeled antibody in order to obtain a detectable and/or quantifiable signal.

The labeled antibodies of the invention, or the functional or fragments of same, include, for example, antibody conjugates (immunoconjugates), which can be combined, for example, with enzymes such as peroxidase, alkaline phosphatase, α-D-galactosidase, glucose oxidase, glucose amylase, carbonic anhydrase, acetylcholinesterase, lysozyme, malate dehydrogenase or glucose-6 phosphate dehydrogenase or by a molecule such as biotin, digoxigenin or 5-bromo-desoxyuridine. Fluorescent labels can be also combined with the antibodies of the invention or functional fragments of same, including notably fluorescein and its derivatives, fluorochrome, rhodamine and its derivatives, green fluorescent protein (GFP), dansyl, umbelliferone, etc. In such conjugates, the antibodies of the invention or functional fragments of same can be prepared by methods known to a person skilled in the art. They can be bound with enzymes or fluorescent labels directly; via a spacer group or a linkage group such as polyaldehyde, glutaraldehyde, ethylenediaminetetraacetic acid (EDTA) or diethylenetriaminepentaacetic acid (DPTA); or in the presence of binding agents such as those mentioned above for therapeutic conjugates. Conjugates carrying fluorescein labels can be prepared by reaction with an isothiocyanate.

Others conjugates can also include chemiluminescent labels such as luminol and dioxetane, bioluminescent labels such as luciferase and luciferin, or radioactive labels such as iodine¹²³, iodine¹²⁵, iodine¹²⁶, iodine¹³³, bromine⁷⁷, technetium^{99m}, indium¹¹¹, indium^{113m}, gallium⁶⁷, gallium⁶⁸, ruthenium⁹⁵, ruthenium⁹⁷, ruthenium¹⁰³, ruthenium¹⁰⁵, mercury¹⁰⁷, mercury²⁰³, rhenium^{99m}, rhenium¹⁰¹, rhenium¹⁰⁵, scandium⁴⁷, tellurium^{121m}, tellurium^{122m}, tellurium^{125m}, thulium¹⁶⁵, thulium¹⁶⁷, thulium¹⁶⁸, fluorine¹⁸, yttrium¹⁹⁹ and iodine¹³¹. Existing methods known to a person skilled in the art for binding radioisotopes with antibodies, either directly or via a chelating agent such as the EDTA or DTPA mentioned above, can be used for as diagnostic radioisotopes. Thus should be mentioned labeling with [I¹²⁵]Na by the chloramine-T technique [Hunter W.M. and Greenwood F.C. (1962) Nature 194:495]; labeling with technetium^{99m} as described by Crockford et al. (US patent 4,424,200) or bound via DTPA as described by Hnatowich (US patent 4,479,930).

The invention also relates to the use of a variant humanized antibody according to the invention for the preparation of a drug for the specific targeting of a compound that is biologically active toward cells expressing or overexpressing JAM-A protein.

In the sense of the present description, a "biologically active compound" is any compound capable of modulating, notably inhibiting, cellular activity, notably growth, proliferation, transcription and gene translation.

The invention also relates to an *in vivo* diagnostic reagent composed of a variant humanized antibody according to the invention, or a derivative or antigen binding fragment of same, preferably labeled, notably radiolabeled, and its use in medical imaging, notably for the detection of cancer related to the cellular expression or overexpression of JAM-A protein.

The invention also relates to a composition as a combination product or to an anti-JAM-A/toxin conjugate or radioisotope, according to the invention, used as drug.

Preferably, said composition as a combination product or said conjugate will be supplemented by an excipient and/or a pharmaceutical vehicle.

In the present description, "pharmaceutical vehicle" means a compound, or a combination of compounds, entering a pharmaceutical composition that does not cause secondary reactions and that, for example, facilitates administration of the active compounds, increases its lifespan and/or effectiveness in the organism, increases its solubility in solution or improves its storage. Such pharmaceutical carriers are well-known and will be adapted by a person skilled in the art according to the nature and the administration route of the active compounds selected.

Preferably, such compounds will be administered by systemic route, notably by intravenous, intramuscular, intradermal, intraperitoneal, subcutaneous or oral route. More preferably, the composition composed of the variant humanized antibody according to the invention will be administered in several doses spaced equally over time.

Their administration routes, dosing schedules and optimal galenic forms can be determined according to the criteria generally taken into account when establishing a treatment suited to a patient such as, for example, the patient's age or body weight, the seriousness of his general state, his tolerance for the treatment and the side effects experienced.

Thus, the invention relates to the use of a variant humanized antibody, or a derivative or antigen binding fragment thereof, for the preparation of a drug for the specific targeting of a compound that is biologically active toward cells expressing or overexpressing JAM-A.

Other characteristics and advantages of the invention appear further in the description with the examples and figures whose legends are presented below.

### FIGURE LEGENDS

Figure 1 shows an alignment between mouse 6F4 VH and each of the selected human V- and J-genes.
Figure 2 shows an alignment between mouse 6F4 VH and each of the selected human V- and J-genes.
Figure 3 illustrates the antibody binding to JAM-A ECD by ELISA. Binding activity of chimeric and humanized antibodies (A: heavy chain; B: light chain) to the JAM-A extracellular domain (ECD) was measured by ELISA where an anti-human Ckappa conjugate was used to detect the purified antibodies. Dose-dependent binding activities onto plastic-coated JAM-A ECD were measured at 450nm.
Figure 4 illustrates the competition binding of biotinylated murine 6F4 to JAM-A ECD by ELISA. Competition binding of biotinylated murine 6F4 Mab to the JAM-A extracellular domain (ECD) by chimeric and humanized (A: heavy chain; B: light chain) antibodies was measured by ELISA. Dose-dependent displacement of biotinylated 6F4 was monitored onto plastic-coated JAM-A ECD.
Figure 5 illustrates the antibody binding and competition binding to JAM-A ECD by ELISA: humanized heavy and light chains. Binding activity (A) of chimeric and humanized Mab to the JAM-A extracellular domain (ECD) was measured by ELISA with an anti-human Ckappa conjugate. Competition binding of biotinylated murine 6F4 Mab (B) to the JAM-A extracellular domain (ECD) by chimeric and humanized antibodies was measured by ELISA.
Figure 6 shows an IEF gel of Hz6F4-1 vs. Hz6F4-2 (2 batches).
Figures 7A and 7B show a Capillary IsoElectric Focusing (cIEF) for Hz6F4-1 (A) and Hz6F4-2 (B).
Figures 8A and 8B show a Cationic Exchange Chromatography (CEX) for Hz6F4-1 (A) and Hz6F4-2 (B).
Figures 9A and 9B show the results of mass spectrometry for the whole antibodies (IgG) for Hz6F4-1 (A) and Hz6F4-2 (B).
Figures 10A and 10B show the results of mass spectrometry for the whole antibodies submitted to PNGase F digestion (IgG) for Hz6F4-1 (A) and Hz6F4-2 (B).
Figures 11A and 11B show the results of mass spectrometry for the heavy chains (HC) for Hz6F4-1 (A) and Hz6F4-2 (B).
Figures 12A and 12B show the results of mass spectrometry for the heavy chains (HC) digested with PNGase F for Hz6F4-1 (A) and Hz6F4-2 (B).
Figure 13 corresponds to a A-431 xenograft tumor model.
Figure 14 corresponds to a DU-145 xenograft tumor model.

### EXAMPLES

### Example 1: Humanization strategy for 6F4 antibody back-up candidates.

In a first step for humanization of the mouse 6F4 monoclonal antibody (Mab), a chimeric form was constructed by fusion of the mouse variable domains, m6F4-VH and m6F4-VL, to respectively human Ckappa and human IGHG4 constant domains. The resulting chimeric antibody belongs to the human IgG4/kappa isotype.

In a next step a first humanized form of the 6F4 Mab was designed based on the selection of a human germline V-gene as close as possible to the mouse V-gene on the basis of amino acid sequence homology. By applying this criteria, IGHV1-f*01 and IGKV1-33*01 were selected for respectively the heavy and light chain acceptor sequences. Nevertheless, the resulting humanized Mab called hz6F4-V1 yielded a calculated isoelectric point (pI) of 5.96 (calculated using the 'iep' application from the wEMBOSS v5.0.0 software) which makes this Mab difficult to be processed by a conventional upstream process purification platform, particularly with regard to the viral inactivation step performed at acidic pH.

Therefore, a novel humanization strategy was designed in which the first selection criteria for the human V-gene acceptor sequence was not exclusively based on amino acid sequence homologies, but rather on the most basic pI, and preferentially a pI value above 7.0 for each VH and VL acceptor V-genes. Definition of framework and CDR domains and selection of human acceptor germline V-genes was performed using the tools and databases of the IMGT website (www.imgt.org). Similarly, during the evaluation of putative back-up mutations in the humanization design, amino acid residues that contain a basic side chain (i.e., arginine, lysine, histidine) will be conserved as much as possible and not considered for back-mutations.

Estimation of a calculated pI value for each humanized, CDR-grafted Mab will be performed using the using the 'iep' application from the wEMBOSS v5.0.0 software.

Humanized variable domains will be cloned into a pConPlus vector carrying either a Ckappa constant domain (pConPlusKappa2, Lonza Biologics, Slough, UK) or a IGHG4 constant domain (pConPlusGamma4PRODeltaK, Lonza Biologics, Slough, UK) and transiently expressed in CHO-K1SV cells. Their ability to bind to the JAM-A target protein will be evaluated by ELISA and compared to a chimeric format of the 6F4 Mab as detailed in the below examples.

### a) Design of humanized 6F4 back-up candidates for the heavy chain variable domain VH.

### - Selection of human acceptor V- and J-genes

A classical database search using the IMGT V-quest tool and based on the nucleotide sequence of the mouse 6F4 VH domain retrieved the IGHV1-f*01 V-gene as best human hit. This human germline V-gene yielded a low pI value of 6.25 and was not selected. In a next step, we applied a database search using the IMGT DomainGapAlign tool and based on the amino acid sequence of the mouse 6F4 VH domain.

**Table 2: Closest human V-genes (DomainGapAlign-based selection)**

| **V gene/allele** | **% identity** | **Overlap** |
|---|---|---|
| IGHV1-3*01 | 64.3 | 98 |
| IGHV1-46*01 | 63.3 | 98 |
| IGHV1-46*03 | 63.3 | 98 |
| IGHV1-2*02 | 63.3 | 98 |
| IGHV1-f*01 | 61.9 | 97 |

**Table 3: Closest human J-genes**

| **V gene/allele** | **% identity** | **Overlap** |
|---|---|---|
| IGHJ4*01 | 86.7 | 15 |
| IGHJ4*02 | 86.7 | 15 |
| IGHJ4*03 | 86.7 | 15 |

### - pI calculation of theoretical, CDR-grafted humanized forms

This calculation is performed by reconstructing theoretical full length heavy chains corresponding to fully humanized forms (each human V-gene carrying the three CDRs of mouse 6F4 and a human IGHJ4*01 J-gene, and an IgG4 constant domain)

**Table 4**

| **V gene/allele** | **Theoretical pI** |
|---|---|
| IGHV1-3*01 | 7.12 |
| IGHV1-2*02 | 7.12 |
| IGHV1-46*01 | 6.90 |
| IGHV1-46*03 | 6.90 |
| IGHV1-f*01 | 6.25 |

Based on both pI calculations and sequence homologies, the selected acceptor human V-and J-genes were IGHV1-3*01 and IGHJ4*01.

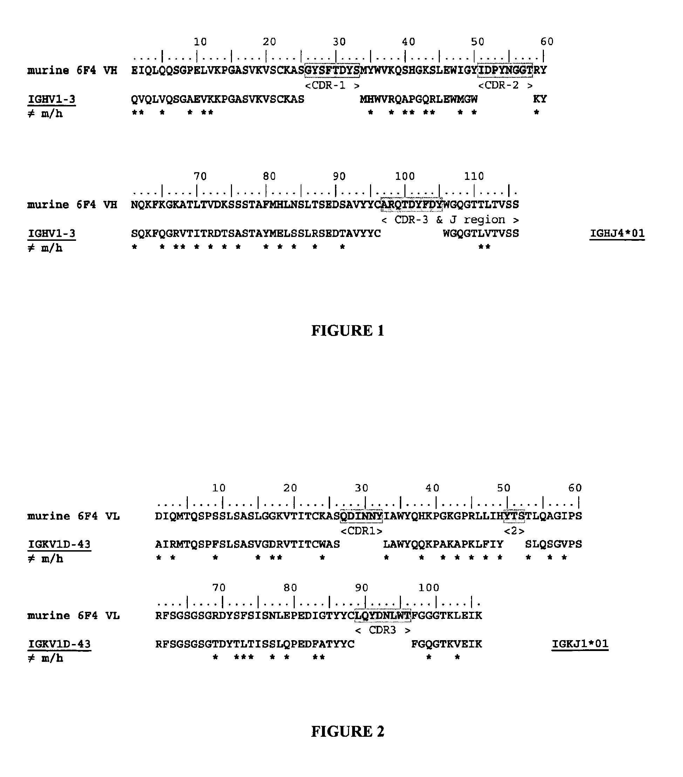

Figure 1 shows an alignment between mouse 6F4 VH and each of the selected human V- and J-genes.

The human IGHV1-3*01 gene has 28 amino acid differences with mouse 6F4 VH, and two additional in the J-region.

Each of these positions will be analyzed individually by taking into account several physico-chemical and structural parameters:
- the isoelectric point of the amino acid. Thirteen residues out of the 30 involve charged amino acids (either basic or acidic). Basic residues such as lysine, histidine or arginine will be conserved as much as possible to keep a global pI above 7.0 ;
- positions as anchors of the CDRs will be conserved as murine in a first instance;
- Vernier zone residues will be considered with high priority.

Based on the above mentioned criteria, one humanized form Hz6F4-2 VH was designed:

Out of the 30 different residues between mouse donor and human acceptor sequences, 21 were directly introduced as human residues and not evaluated as back-mutations due to their non critical position in term of VH structure. They also favor the introduction of basic residues such as Lys 12, His35 and Arg87.

### b) Design of humanized 6F4 back-up candidates for the light chain variable domain VL.

### - Selection of human acceptor V- and J-genes

A classical database search using the IMGT V-Quest tool and based on the nucleotide sequence of the mouse 6F4 VL domain retrieved novel putative human germline V-gene listed below (best hits) and their percentage of identity both at the nucleotide and amino acid levels.

**Table 5: Closest human V-genes (V-Quest-based selection)**

| **V gene/allele** | **% identity (nucleotide)** | **% identity (aminoacid)** | **Overlap** |
|---|---|---|---|
| IGKV1-33*01 | 81 | 73.4 | 94 |
| IGKV1-27*01 | 79 | 69.9 | 93 |
| IGHV1-D-43*01 | 78 | 67.8 | 90 |
| IGHV1-NL*01 | 78 | 67.3 | 89 |

Note: the IGKV1-33 V-gene was selected as human acceptor sequence for the first humanization strategy. It was not selected for the below described design. A V-gene ranking based on the DomainGapAlign tool gave the same list of closest human IGKV genes.

**Table 6: Closest human J-gene**

| **V gene/allele** | **% identity (nucleotide)** | **% identity (aminoacid)** | **Overlap** |
|---|---|---|---|
| IGKJ1*01 | 86.5 | 83.3 | 12 |

### - pI calculation of theoretical, CDR-grafted humanized forms

This calculation is performed by reconstructing theoretical full length light chains corresponding to fully humanized forms (each human V-gene carrying the three CDRs of mouse 6F4 and a human IGKJ1*01 J-gene, and a human Ckappa constant domain)

**Table 7**

| **V gene/allele** | **Theoretical pI** |
|---|---|
| IGKV1-33*01 | 5.50 |
| IGKV1-27*01 | 6.16 |
| IGHV1-D-43*01 | 6.89 |
| IGHV1-NL*01 | 6.19 |

Based on both pI calculations and sequence homologies, the selected acceptor human V-and J-genes were IGKV1D-43*01 and IGKJ1*01.

Figure 2 shows an alignment between mouse 6F4 VH and each of the selected human V- and J-genes.

The human IGKV1D-43*01 gene has 25 amino acid differences with mouse 6F4 VL and two additional in the J-region.

Each of these positions will be analyzed individually by taking into account several physico-chemical and structural parameters:
- the isoelectric point of the amino acid. Eight residues involve charged amino acids (either basic or acidic). Basic residues such as lysine, histidine or arginine will be conserved as much as possible to keep a global pI above 7.0 ;
- positions as anchors of the CDRs will be conserved as murine in a first instance;
- Vernier zone residues will be considered with high priority.

Based on the above mentioned criteria, one humanized form Hz6F4-2 VL was designed.

Out of the 27 different residues between mouse donor and human acceptor sequences, 21 were directly introduced as human residues and not evaluated as back-mutations due to their non critical position in term of VL structure. The CDR2 anchors His49 and Thr53 were conserved as their murine counterpart due to their strong implication in CDR structure and basic side chain (His).

### Example 2: Production of chimeric and humanized 6F4 Mabs

All Mab were produced upon transient transfection and by using the CHO-K1SV system with pConPlusKappa2 (light chain) and pConPlusGamma4PRODeltaK (heavy chain) expression vectors (Lonza Biologics, Slough, UK).

The entire nucleotide sequences corresponding to the humanized versions of the variable domain of 6F4 Mab light and heavy chains were synthesized by global gene synthesis (Genecust, Luxembourg). They were subcloned into pConPlusKappa2 (light chain) or pConPlusGamma4PRODeltaK (heavy chain) expression vectors (Lonza Biologics, Slough, UK) carrying the entire coding sequence of the corresponding constant domain. All cloning steps were performed according to conventional molecular biology techniques as described in the Laboratory manual (Sambrook and Russel, 2001) or according to the supplier's instructions. Each genetic construct was fully validated by nucleotide sequencing using Big Dye terminator cycle sequencing kit (Applied Biosystems, US) and analyzed using a 3100 Genetic Analyzer (Applied Biosystems, US).

Suspension-adapted CHO-K1SV cells (Lonza Biologics, Slough) were routinely grown in 250 ml flasks in 50 ml of chemically-defined, serum free CD CHO culture medium (In Vitrogen, US) on an orbital shaker. Transient transfection was performed by electroporation using a GenePulser apparatus (BioRad Laboratories, US) and 10⁷ cells and 80 µg of expression vectors in a 2:1 ratio for H/L chain vectors in a 600 µl volume. The transfection mix was used to inoculate a 30-50 ml culture in CD-CHO medium. Cultivation process was monitored on the basis of cell viability and Mab production. Typically, cultures were maintained for 5 to 7 days. Mabs were purified using a conventional chromatography approach on a Protein A resin (GE Healthcare, US).

Antibody forms were produced at levels suitable with ELISA evaluations. Productivity levels are typically ranging between 1 and 5 mg/l of purified Mabs.

### Example 3: Evaluation of JAM-A binding capacity of chimeric and humanized Mabs by ELISA

A first screening assay corresponds to the ability of the chimeric and humanized Mabs to bind to the JAM-A extracellular domain.

Briefly, ELISA plates (Immulon II HB, VWR) were coated overnight at 4°C with a recombinant protein [4 µg/ml] corresponding to the JAM-A extracellular domain and produced in E.coli and saturated with 1% bovine serum albumine (BSA) in PBS. After three washes with PBS containing 0.05 % of Tween20, samples to analyze (200 µl) were serially diluted, added to the microtiter plate and incubated for 1H at 37°C. After three washes, a detection antibody corresponding to an anti-human C-kappa antibody coupled to peroxidase (Ref. A7164, Sigma Aldrich) was added at a dilution of 1/5000 and incubated for 1 H at 37°C. After three additional washes, a peroxidase colorimetric substrate was added (ELISA peroxidase substrate, Interchim) and incubated for 5 min. at room temperature.

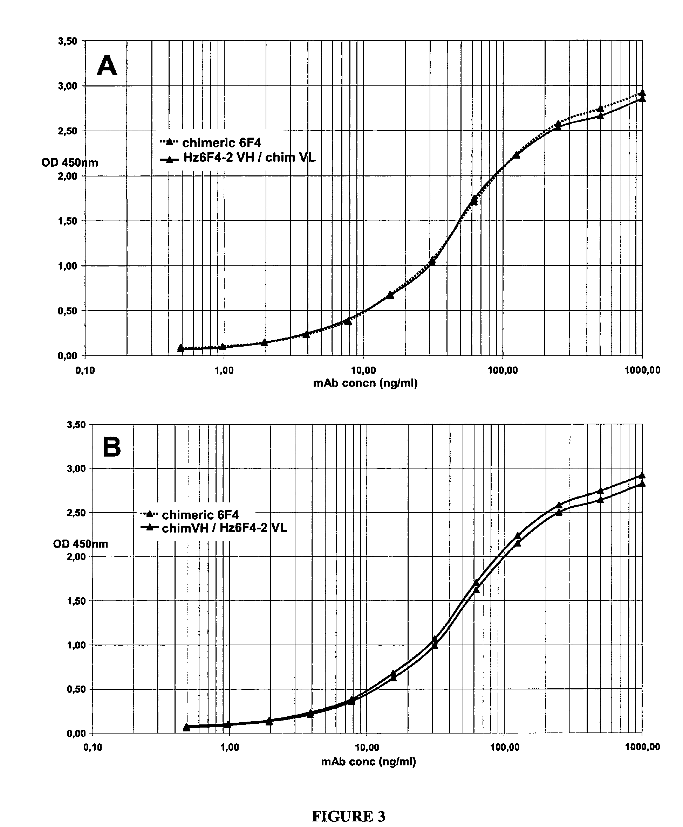

Figure 3A shows that the binding capacity of the humanized heavy chain Hz6F4-2 VH combined with a chimeric 6F4 Mab light chain was almost identical to that of a chimeric 6F4 Mab. Similar results were obtained with the humanized light chain Hz6F4-2 VL combined with a chimeric heavy chain (Figure 3B).

### Example 4: Evaluation of the capacity of humanized Mabs to compete with biotinylated murine 6F4 Mab for binding to JAM-A

A second screening assay corresponds to the ability of the chimeric and humanized Mabs to compete with biotinylated mouse 6F4 Mab for binding to the JAM-A extracellular domain.

* *6F4 Mab biotinylation procedure* : 6F4 Mab was labeled with biotin (sulfo-NHS-LC-biotin, Perbio Science - 1 mg/ml in 0.1 M sodium bicarbonate buffer - dye-to-antibody ratio of 20/1, mol:mol) and the mixture was incubated in the dark for 30 min at room temperature. The reaction was immediately dialyzed overnight at 4°C in the dark against PBS.

Protein concentration of the labeled Mab was determined by BCA assay. It was also characterized by SDS-PAGE electrophoresis under reducing and non reducing conditions. No difference with the non-labeled m6F4 Mab could be observed. Biotin labeling was further assessed by Western blot: integrity of the labeled Mab solution was confirmed and showed that biotin was equivalently coupled to both heavy and light chains.

* *ELISA procedure* : ELISA plates (Immulon II HB, VWR) were coated overnight at 4°C with a recombinant protein [4 µg/ml] corresponding to the JAM-A extracellular domain and produced in E.coli and saturated with 1% bovine serum albumine (BSA) in PBS. After three washes with PBS containing 0.05 % of Tween20, samples to analyze (200 µl) were serially diluted and added to the microtiter plate together with a fixed concentration of biotinylated mouse 6F4 Mab [biot-m6F4: 2 µg/ml]. Incubation was performed for 1H at 37°C. After three washes, a detection antibody corresponding to Avidin-coupled peroxidase (Ref. A3151-1MG, Sigma Aldrich) was added at a dilution of 1/2000 and incubated for 1 H at 37°C. After three additional washes, a peroxidase colorimetric substrate was added (ELISA peroxidase substrate, Interchim) and incubated for 5 min. at room temperature.

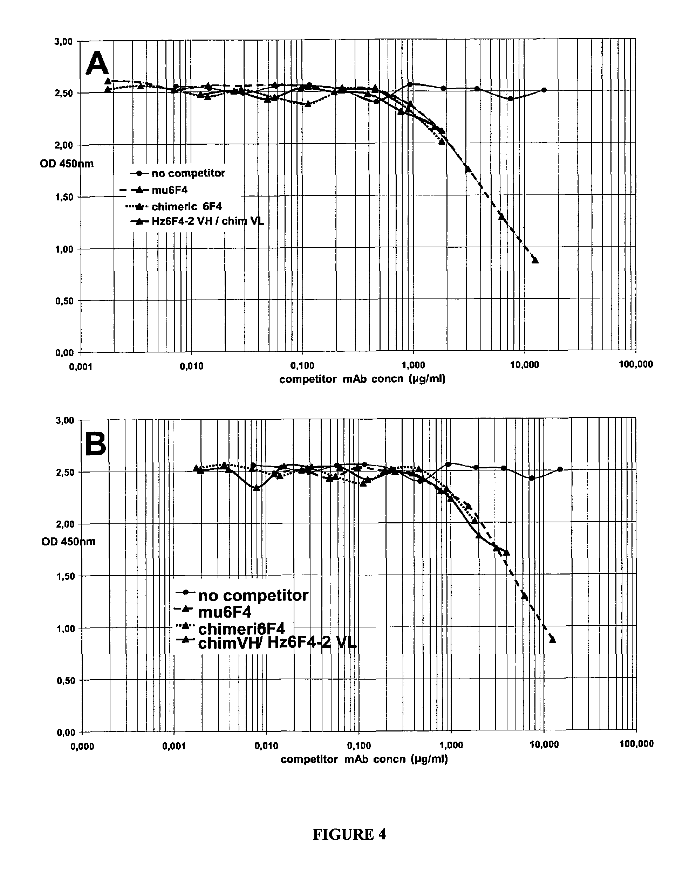

Figure 2 shows the competition binding capacity of the humanized heavy and light chains combined with a chimeric 6F4 Mab counterpart. Hz6F4-2 VH (Figure 4A) and Hz6F4-2 VL (Figure 4B) yielded potent and efficacious capacity to inhibit biot-m6F4 binding to JAM-A ECD.

### Example 5: Evaluation of the capacity of humanized Mabs to compete with biotinylated murine 6F4 Mab for binding to JAM-A

Hz6F4-2 VH was combined with Hz6F4-2 VL chain and the resulting antibody was evaluated for its capacity to bind to JAM-A ECD and to displace bio-m6F4 from its target antigen, as described in the above examples.

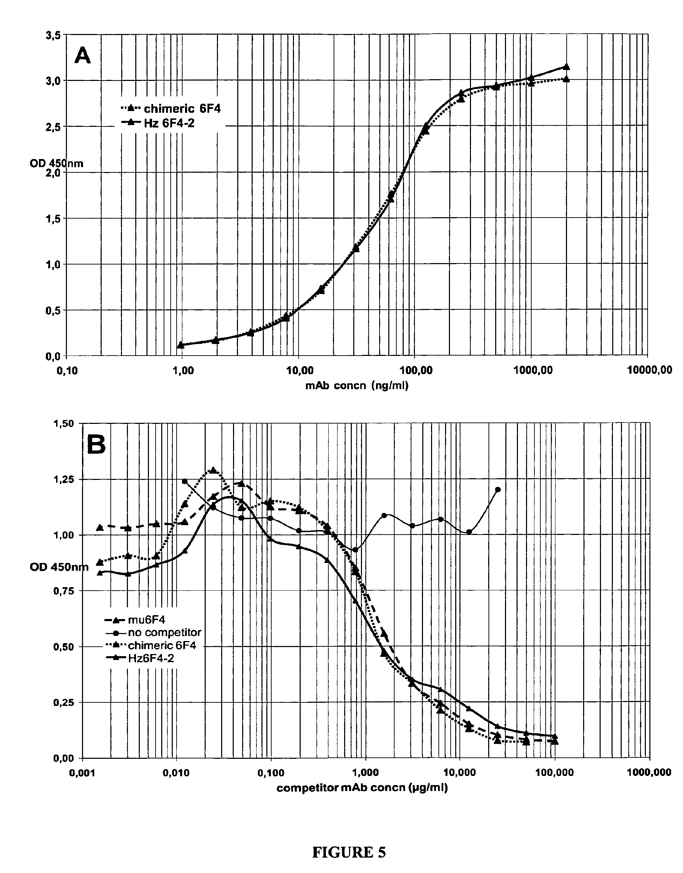

Figure 5A shows the ability of the fully humanized Mab to recognize JAM-A ECD. Hz6F4-2 yielded a binding capacity to the extracellular domain of JAM-A almost identical to a chimeric 6F4 Mab.

Figure 5B shows the competition binding capacity of the fully humanized Mab to inhibit biot-m6F4 binding to JAM-A ECD. Hz6F4-2 was a potent and efficacious competitor of biot-m6F4 binding to JAM-A ECD.

### Example 6: IsoElectric Focusing (IEF): Hz6F4-1 and Hz6F4-2

Samples are analyzed by IEF (3-10) on AMERSHAM Multiphor II electrophoresis unit.

Dry gel is rehydrated with 16.5 ml buffer made with 1.65 g sorbitol, 1230 µl pharmalytes pH 3-10 and water. After sonication, the rehydratation solution is put into the GelPool and the gel is place on with the surface downwards. After 2 hours it can be removed and be used at once.

Connect the Multiphor II electrophoresis unit to cryostat and set the temperature to 10°C 15 minutes before starting the analysis. Put 5 ml of glycerol anhydre onto the cooling plate of Multiphor II and position the gel, take care no air bubble trapped beneath.

Position sample application pieces and depose 15 µl (5 µg) of sample concentration 0.33 mg/ml and 15 µl standards of pI 5-10.5.

The migration is executed on 3 hours, 0V to 2000 V, with a break at half time to remove the sample application pieces. Gel is colored with Coomassie blue and dry with a cellophane sheet. Gel is scanned and pI are determined with the logiciel "Quantity One"

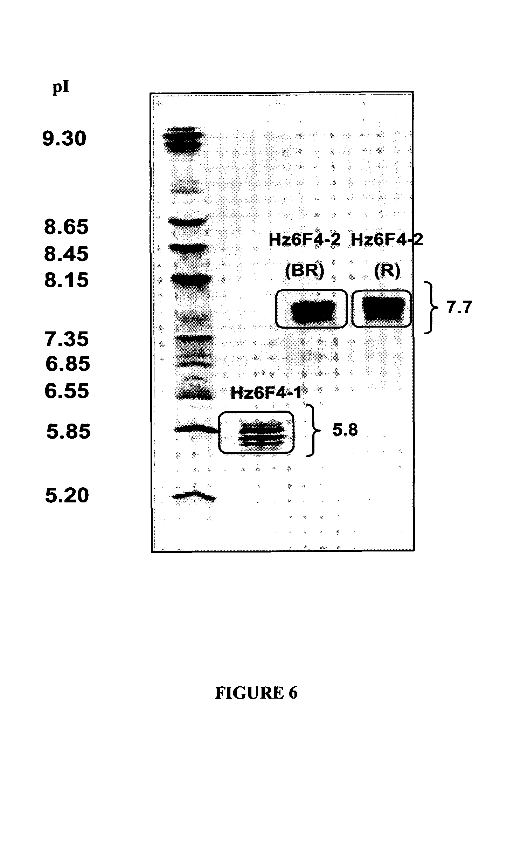

As illustrated by Figure 6, the obtained IEF gel of Hz6F4-1 vs. Hz6F4-2 (2 batches) shows both a near 2 units increase of pI (5.8 vs. 7.7) and a significant reduced number of band for Hz6F4-2.

### Example 7: Capillary IsoElectric Focusing (cIEF): Hz6F4-1 and Hz6F4-2

A feasibility study was realized by BECKMAN with Hz6F4-1 and Hz6F4-2. The iso-electric point (pI) determination was made on a ProteomLab PA800 of BECKMAN instruments. An eCAP Neutral capillary of 30 cm length and 50 µm I.D. diameters was used, at 20°C temperature with an aperture 100 x 200µm.

Analyte (Phosphoric acid 200 mM), catholyte (Sodium hydroxide 300 mM), chemical mobilizer (Acetic acid 350mM), cathodic stabilizer (L-Arginine, 500 mM), anodic stabilizer (Iminodiacetic acid, 200 mM), Urea solution (Urea, 4,3M) and 3M Urea-cIEF gel solutions were prepared.

Analyte solution is composed of H3PO4 at 200 mM in water. Catholyte solution is composed by NaOH at 300 mM in water. Chemical Mobilizer is composed by CH₃COOH at 350mM in water. Cathodic stabilizer is composed by L-arginine at 500mM in water. Anodic stabilizer is composed by Iminodiacetic acid at 200mM in water. 3M Urea-cIEF gel solution is composed by urea 3M in cIEF gel kit.

Urea solution was used during 5 minutes at 50 psi to rinse capillary between each analysis.

A master mix solution was prepared with 200 µL of 3M Urea-cIEF gel, 12 µL of Pharmalyte pH 3-10 (GE Healthcare, ref 17-0456), 20 µL of cathodic stabilizer, 2 µL of anodic stabilizer and pI markers (2µl of peptide pI 10; 2 µl peptide pI 9,5 and 2 µL of peptide pI 4,1).

The protein sample was buffer exchanged with Tris 20mM solution on a microcon YM-30 because the protein sample must have a salt concentration below 50mM.

After rinsing capillary with Urea solution at 4,3 mM and H2O solution during 2 min, the sample was injected to anode side. A focusing step is made at 25 kV during 15 min then mobilization at 30 kV during 30 min. Detection was performed at 280 nm and data were analyzed using Beckman 32Karat 5.0 software.

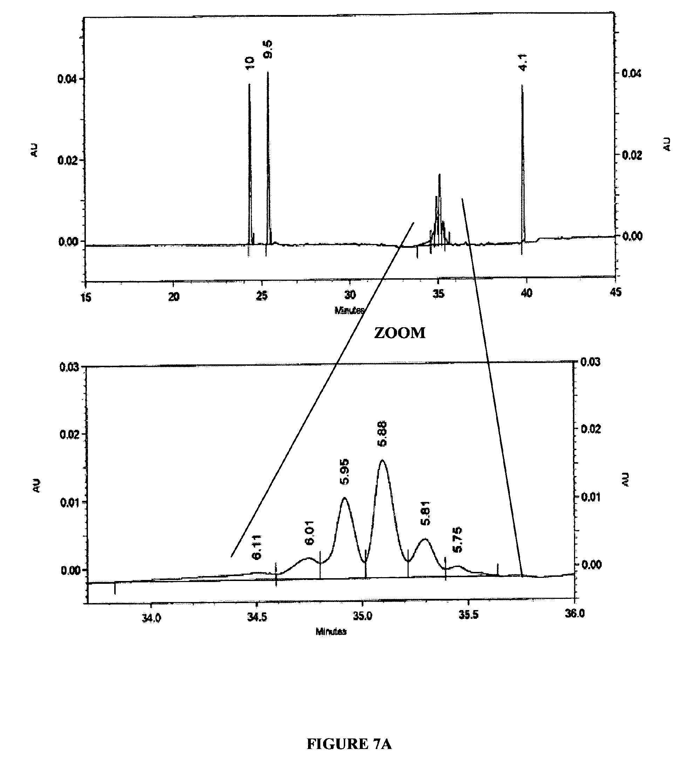

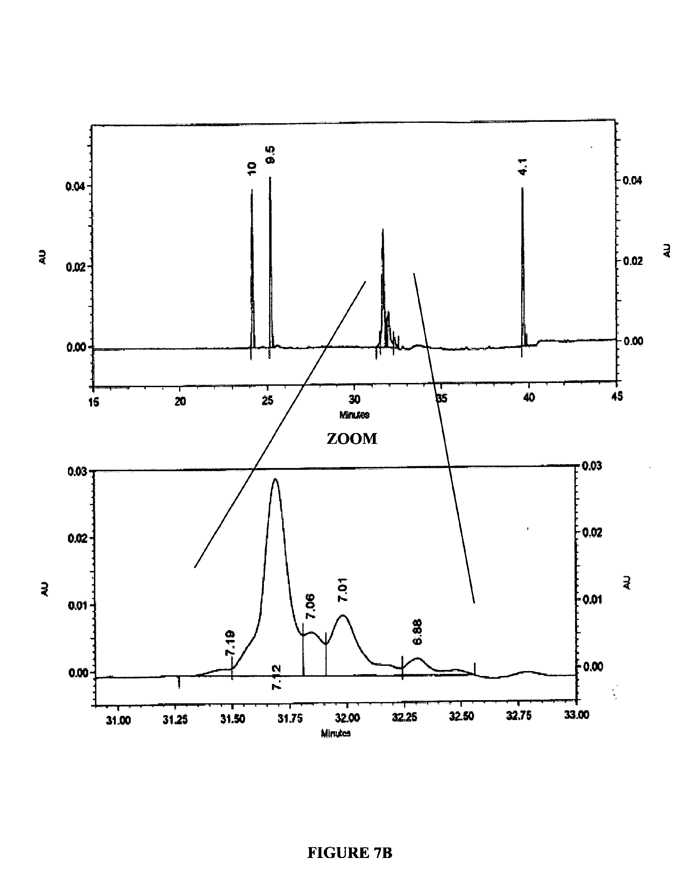

Figures 7A and 7B show the data of a cIEF experiment. Again Hz6F4-2 is clearly more homogeneous than Hz6F4-1.

### Example 8: Cationic Exchange Chromatography: Hz6F4-1 and Hz6F4-2

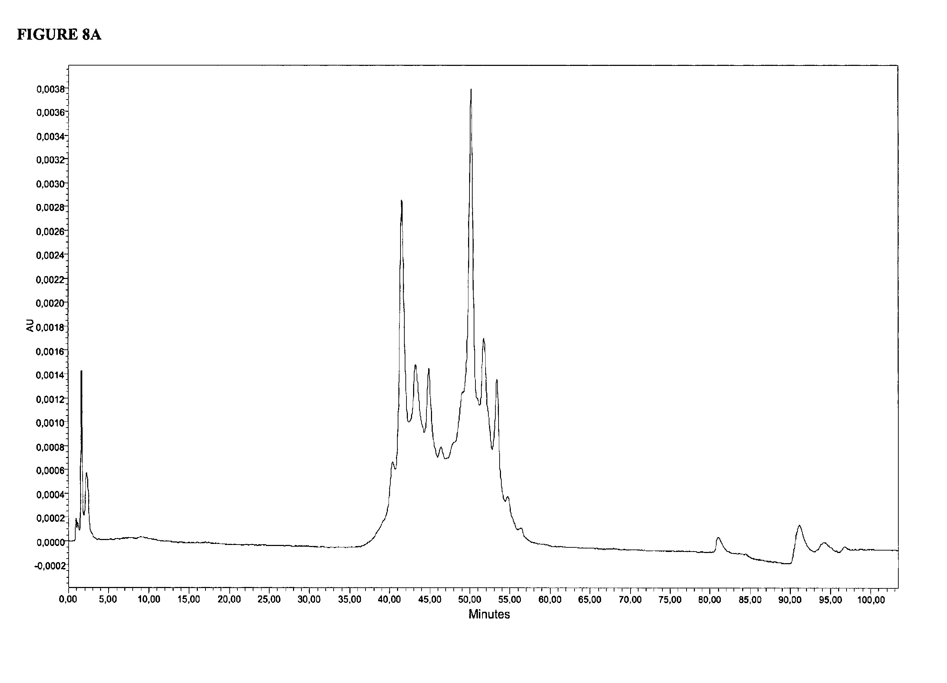

### a) Cationic Exchange Chromatography: Hz6F4-1 (Figure 8A)

The different isoforms of antibodies were separated on a Dionex WCX-10 cation exchange column, 4 x 250 mm with a WCX-10 guard column 4 x 50 mm on a Waters HPLC system, consisted of two 515 pumps, a 717 autoinjector and a 2487 UV detector. Buffer A was 20 mM sodium acetate, pH 5.0, and buffer B was 20 mM sodium acetate and 1 M NaCl , pH 5.0. The flow rate was 1.0 ml/min. The amount injection was approximately 20 µg. The gradient for elution was 0 min at 100% Solvent A followed by a linear gradient of 25% solvent B over 77.5 min. After elution, the column was washed with 75% solvent B for 9 min and reequilibrated with 100% solvent A for 25 min. The elution was monitored by UV spectroscopy at 280 nm.

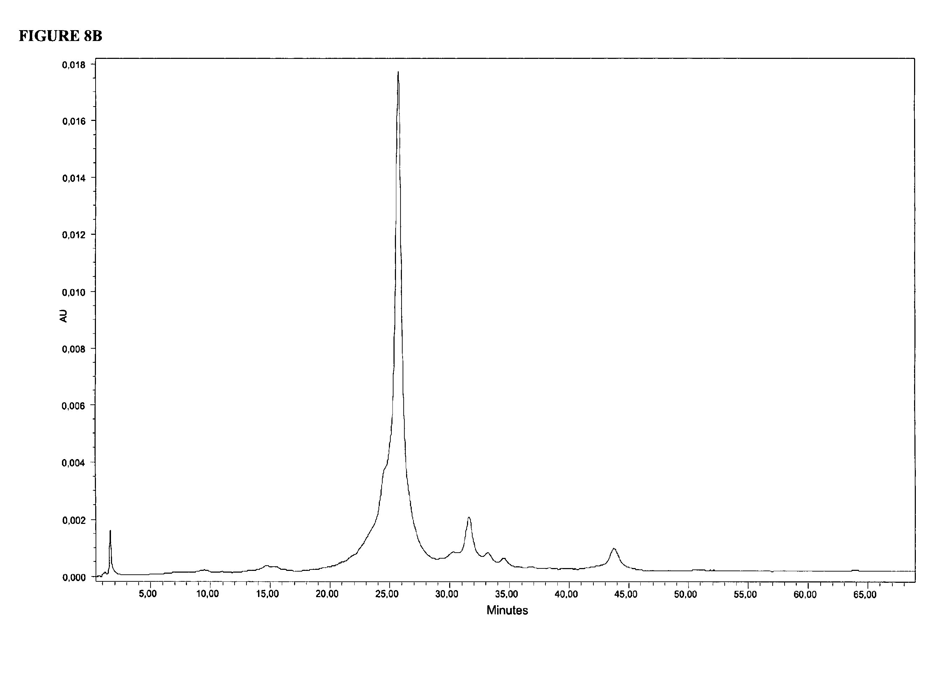

### b) Cationic Exchange Chromatography: Hz6F4-2 (Figure 8B)

The different isoforms of antibodies were separated on a Dionex WCX-10 cation exchange column, 4 x 250 mm with a WCX-10 guard column 4 x 50 mm on a Waters HPLC system, consisted of two 515 pumps, a 717 autoinjector and a 2487 UV detector. Buffer A was 20 mM sodium phosphate, pH 6.0, and buffer B was 20 mM sodium phosphate and 1 M NaCl , pH 6.0. The flow rate was 1.0 ml/min. The amount injection was approximately 20 µg. The gradient for elution was 0 min at 4% Solvent B followed by a linear gradient of 4-15% solvent B over 73 min. After elution, the column was washed with 75% solvent B for 9 min and re-equilibrated with 96% solvent A for 25 min. The elution was monitored by UV spectroscopy at 280 nm.

Figures 8A and 8B show the data of a CEX analysis. Hz6F4-2 is clearly more homogeneous than Hz6F4-1.

### Example 9: Mass Spectrometry: whole antibody without and with PNGase F treatment

### - Equipments :

- LCT Premier Waters (SM04) mass spectrometer equipped with Masslynx^{™} 4.1, Transform^{™} 4.1, MaxEnt^{™} 4.1, Biolynx^{™} 4.1
- Alliance HPLC (CH07)
- UV detector (PDA) DT 0003
- Desalting column: Mass Prep Online Desalting Cartridge (2.1x10 mm, Waters)
- 2 mL vials (Waters, WAT270946C) and 150 µL inserts (Waters, ref: WAT094171DV)
- 250 µL syringe (Hamilton, GasTight 1725RNR, ref: 81165)
- 0.5 mL Eppendorf tubes
- Pipets and corresponding tips
- Thermomixer + block tube 0.5 mL

### - Chemicals/Reagents:

- Preparation of HPLC eluting solutions:
   ○ Acetonitril (Carlo Erba, ref P00637521 or similar)
   ○ Formic Acid (FA, Sigma/Fluka, ref: 06440 or similar)
   ○ MilliQ water
- Isopropanol (Isopropylic Alcohol, Carlo Erba, ref: 412712 or similar)
- PeptideN-Glycosidase (PNGase F, BioLabs New England ref: P0704L / 75 000 units or similar)
- Cesium Iodide (CsI, Merck, ref: 102861 or similar)
- Reference mAb: B6AHM or similar

### - Sample preparation:

The intact non deglycosylated mAb was injected without any prior treatment. For the analysis take a sample volume to have 15 µg and injected 10 µg. For the LCMS analysis used the desalting column Mass Prep Online Desalting Cartridge (2.1x10mm, Waters) with a flow rate 0.4mL/min. The HPLC eluting solutions were acetonitril 0.1% formic acid for solvent B and water 0.1% acid formic for solvent A. The flow rate was 0.4mL/min and the oven temperature was 60°C. The gradient for elution was 1min at 95% solventA, 3min30 at 80% solvent B and 2min30 at 95% solvent A. Mass spectrometry was performed on the LCT Premier with a capillary at 3400V, the desolvation temperature 350°C, source temperature 150V and sample cone at 120V.

For deglycosylated sample, take 100 µg of Mab in 0.5mL Eppendorf tube. Add 0.5µL of PNGase F and incubate overnight in the thermomixer at 37°C and mixed (800t/min). For the analysis take a sample volume to have 15µg and injected 10µg. For the LCMS analysis used the desalting column Mass Prep Online Desalting Cartridge (2.1x10mm, Waters) with a flow rate 0.4mL/min. The HPLC eluting solutions were acetonitril 0.1 % formic acid for solvent B and waters 0.1 % acid formic for solvent A. The flow rate was 0.4mL/min and the oven temperature was 60°C. The gradient for elution was 1min at 95% solventA, 3min30 at 80% solventB and 2min30 at 95% solventA. Mass spectrometry was performed on the LCT Premier (Micromass Waters) with a capillary at 3,400 V, the desolvation temperature 350°C, source temperature 150 V and sample cone at 120 V.

### - Results:

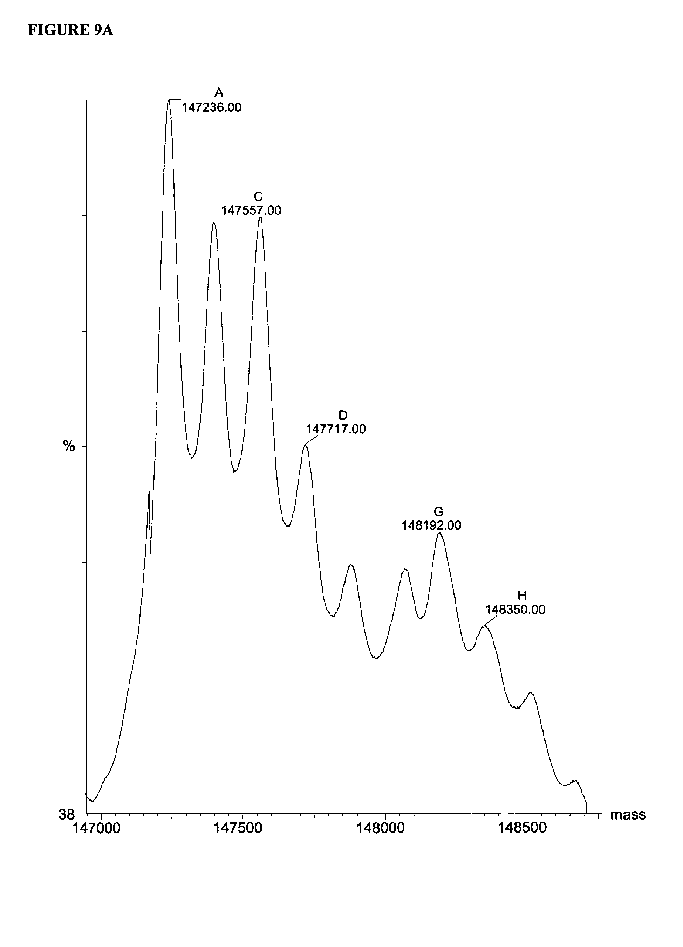

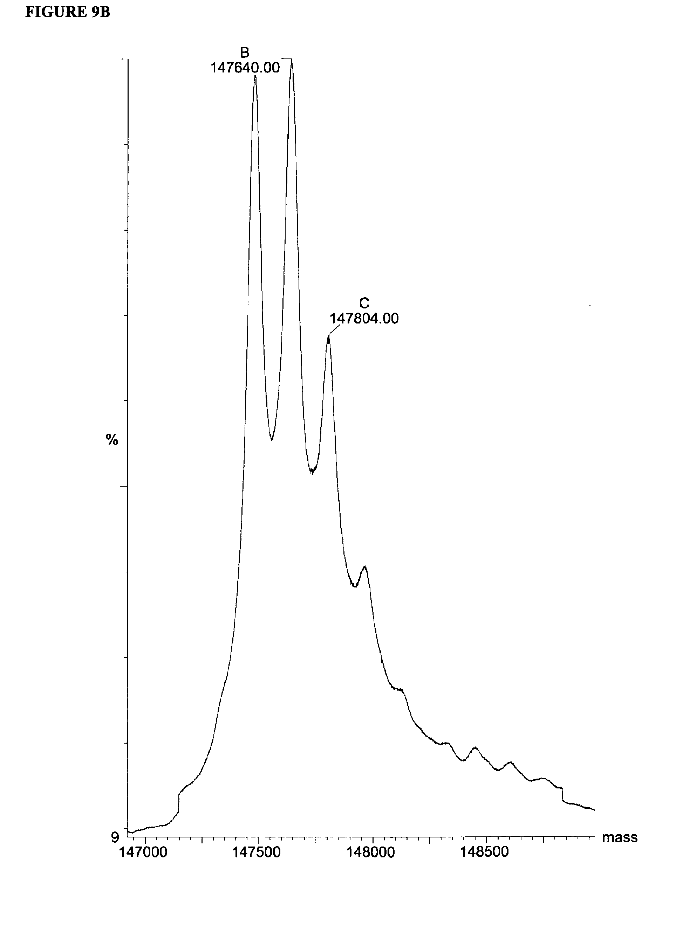

For the whole antibodies (IgG), Figures 9A and 9B show the data of a LC-ESI-TOF experiment. Hz6F4-2 (Figure 9B) is clearly more homogeneous than Hz6F4-1 (Figure 9A). Hz6F4-2 shows four main peaks corresponding to expected IgG + main glycoforms (eg: G0/G0F, G0F/G1F, G1F/G1F, G1F/G2F). Hz6F4-1 shows also 4 main peaks corresponding to expected IgG + glycoforms (eg: G0/G0F, G0F/G1F, G1F/G1F, G1F/G2F) but with high amount of additional impurities.

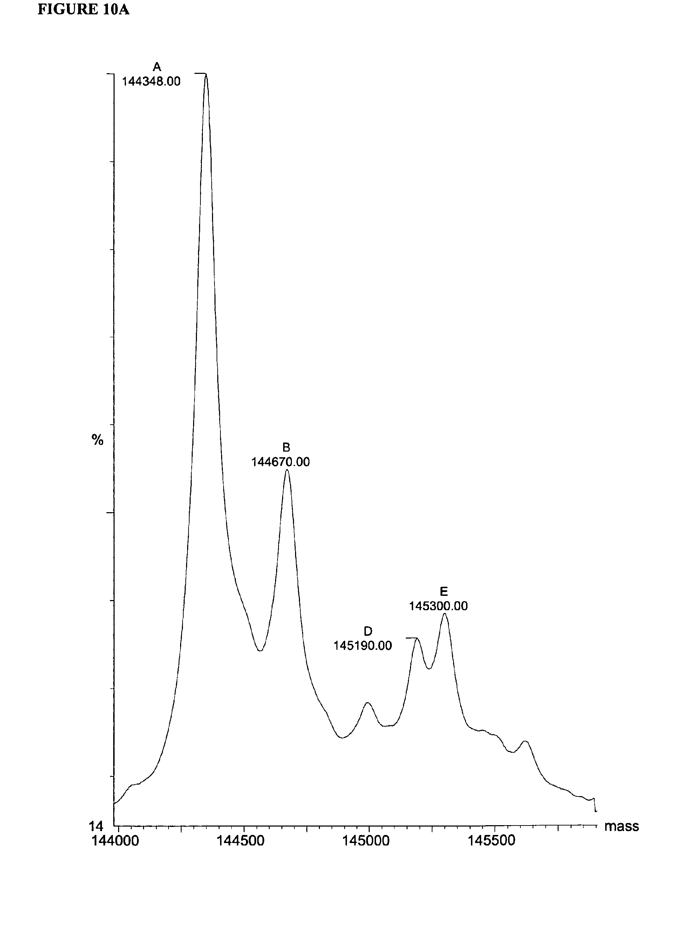

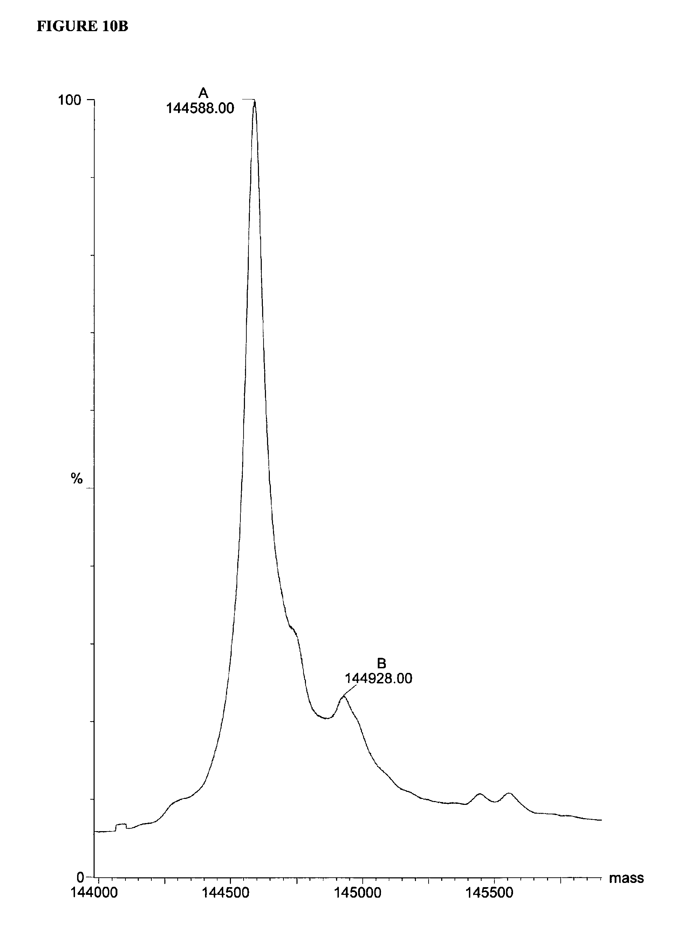

For the whole antibodies submitted to PNGase F digestion (IgG), Figures 10A and 10B show the data of a LC-ESI-TOF experiment. Hz6F4-2 (Figure 10B) is clearly more homogeneous than Hz6F4-1 (Figure 10A). After PNGase F digestion, Hz6F4-2 shows a main peak corresponding to expected non de-glycosylated IgG a minor peak is also visible that may correspond to 2 glycationsd sites (+ 340 Da). Conversely Hz6F4-1 shows alos main peak corresponding to expected de-glycosylated IgG but with very high amount of at least 5 additional impurities of higher mass.

### Example 10: Mass Spectrometry: reduced antibody (Light and Heavy Chains) without and with PNGase F treatment

### - Equipments :

- LCT Premier Waters (SM04) mass spectrometer equipped with Masslynx^{™} 4.1, Transform^{™} 4.1, MaxEnt^{™} 4.1, Biolynx^{™} 4.1
- Alliance HPLC (CH07)
- UV detector (PDA) (DT 0003)
- Column X BridgeBEH 300 C4 2,1x150 mm, 3,5 µm (Waters PN 186004500 LN 0104191601)
- 2 mL vials (Waters, WAT270946C) and 150 µL inserts (Waters, ref WAT094171DV)
- 250 µL syringe (Hamilton, GasTight 1725RNR, ref 81165)
- 0.5 mL Eppendorf tubes
- Pipets and corresponding tips
- Thermomixer + block tube 0,5 mL
- Lyophilisator (LY01).

### - Chemicals/Reagents :

- Preparation of HPLC eluting solutions:
   ○ n-propanol (n-propyl alcohol, Carlo-Erba, ref: 412542 or similar)
   ○ Formic Acid (FA, Sigma/Fluka, ref: 06440 or similar)
   ○ Trifluoroacetic Acid (TFA, Fluka, ref: 91699 or similar)
   ○ MilliQ water
- Reduction buffer preparation :
   ○ Tris HCl (Trizma Base, Sigma, ref: T6066 or similar)
   ○ Ethylenediamine tetraacetic acid (EDTA, ref: ED2SS or similar)
   ○ Guanidine chlorhydrate (Guanidine hydrochlorid 99%, Aldrich, ref: 177253 or similar)
   ○ MilliQ water
   ○ Chlorhydric acid 6N (HCl, Merck, ref: 100314 or similar)
   ○ Dithiothreitol (DTT, Aldrich, ref: 150460 or similar)
- Isopropanol (Isopropylic Alcohol, Carlo Erba, ref: 412712 or similar)

### - Sample preparation:

For the non deglycosylated reduced mAb, lyophilized a mAb sample volume corresponding to 100 µg in 0.5mL Eppendorf. Solubilized in 20µl reduction buffer (0.121 g Tris HCl, 7.4 mg EDTA, 5.73 g Guanidine HCl, solubilise in 9ml with MilliQ water, adjust the pH at 8.0 with HCL 6N and complete to 10 mL with MilliQ water). 30s under nitrogen flow, added 0.8 µL of 80 mg/mL DTT solution and incubated 2H in thermomixer at 37°C under mixing (800 t/min). Quenched the reaction by adding 2µL acetic acid and 4 µL n-propanol. Just before injection add 73.2µL MilliQ water.

For the deglycosylated mAb take a sample volume to have 100µg in 0.5mL Eppendorf. Lyophilized and then solubilised in 20µL reduction buffer (0.121g Tris HCL, 7.4 mg EDTA, 5.73 g Guanidine HCl, solubilised in 9ml with MilliQ water, adjusted the pH at 8.0 with HCl 6N and completed to 10 mL with MilliQ water). 30 s under nitrogen flow, added 0.8 µL of 80 mg/mL DTT solution and incubated 2H in thermomixer at 37°C under mixing (800 t/min). Quenched the reaction by adding 2µL acetic acid and 4 µL n-propanol. Just before injection add 73.2 µL MilliQ water.

The sample analysis was performed on the Alliance HPLC coupled to mass spectrometer LCT Premier (Micromass Waters). For the LCMS analysis used the Xbridge column BEH300 C4 (2.1x10 mm,Waters) with a flow rate 0.25 mL/min. The HPLC eluting solutions were water 0.05% trifluoroacetic acid for solvent A and n-propyl alcohol/water/formic acid/trifluoroacetic acid (90/10/0.1/0.02) for solvent B. The flow rate was 0.25mL/min and the oven temperature was 60°C. The gradient for elution was 10min 95% solvent A to 80% with slope 1.5%/min, 30 min at 20% solvent B to 50% with slope 1%/min, and 5min at 80% solvent B then returned to the initial conditions at 95% solvent A. Mass spectrometry was performed on the LCT Premier (Micromass Waters) with a capillary at 3,400 V, the desolvation temperature 350°C, source temperature 150 V and sample cone at 120 V.

### - Results:

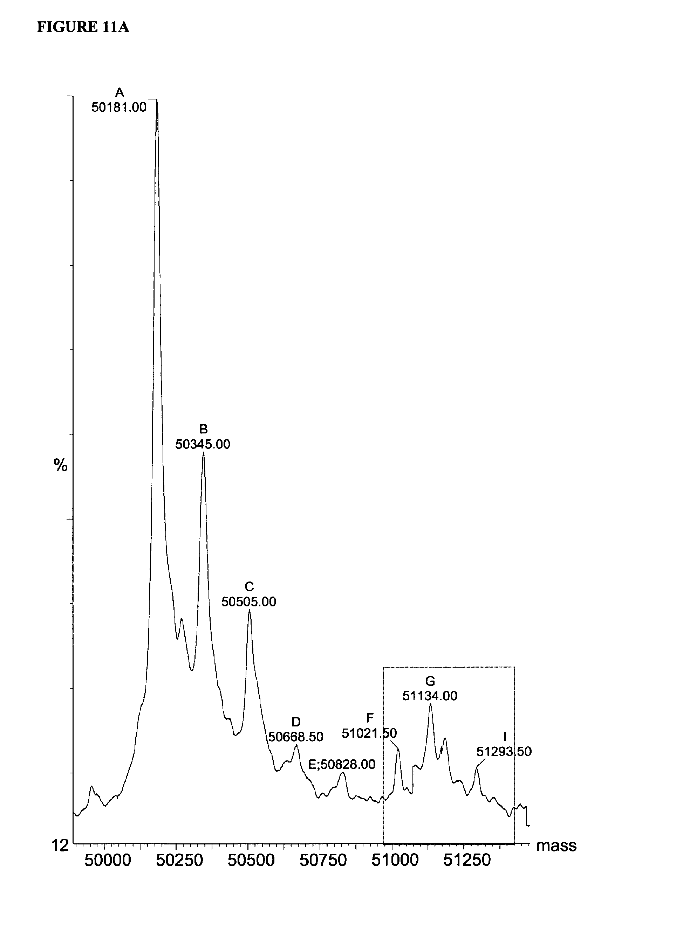

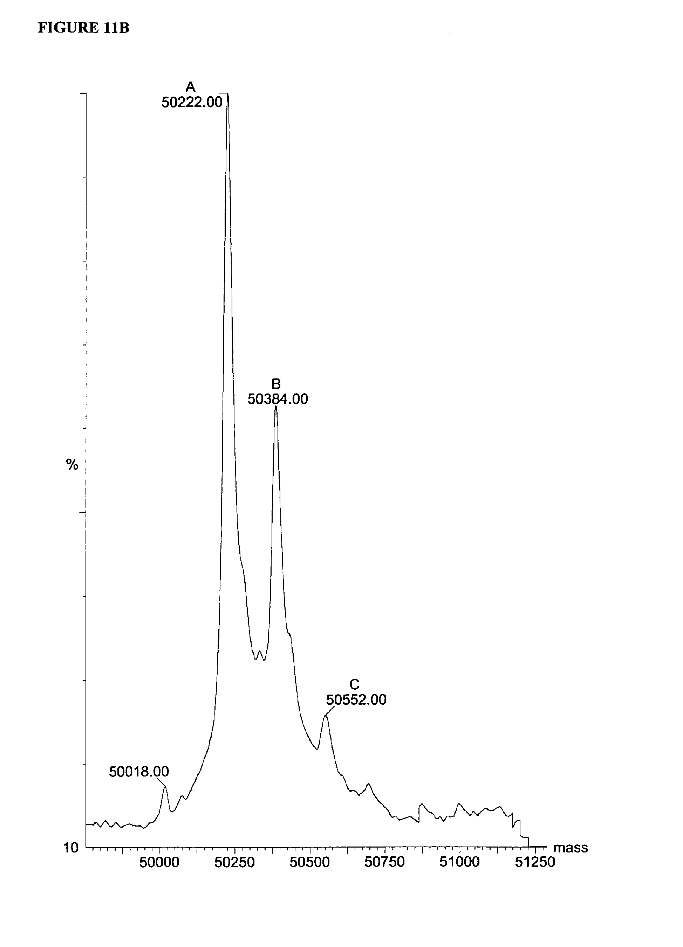

For the heavy chains (HC), Figures 11A and 11B show the heavy chain of Hz6F4-1 (Figure 11A) and Hz6F4-2 (Figure 11B). In both the cases the 3 main peaks have experimental masses in agreement with the theoretical polypeptide sequence plus the expected glycoforms (GOF, G1F and G2F, respectively). Nevertheless, the heavy chain of Hz6F4-1 shows additional peaks that confirm the higher heterogeneity in comparison to Hz6F4-2.

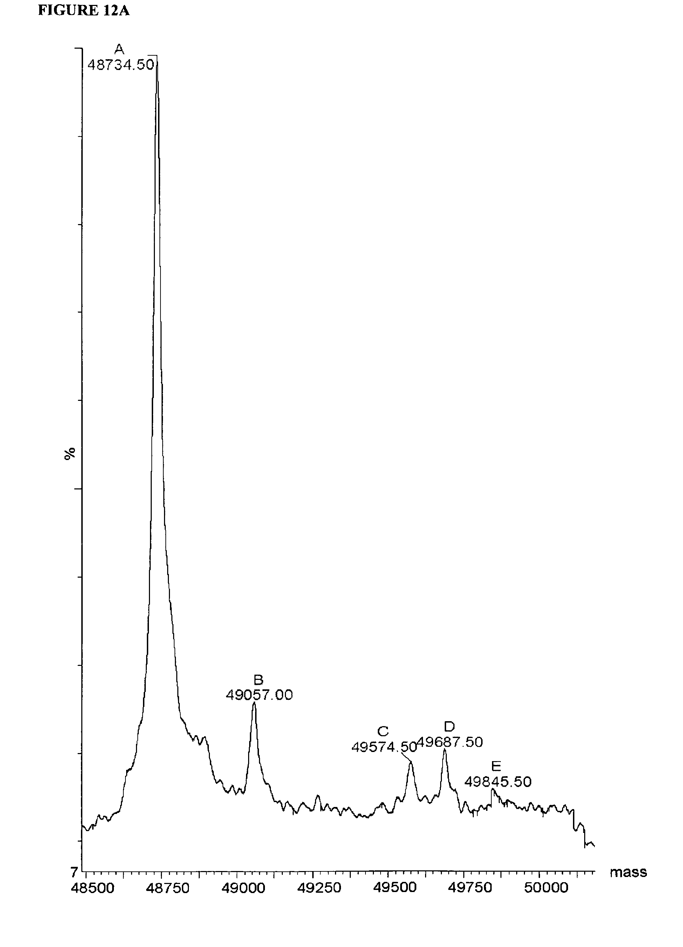

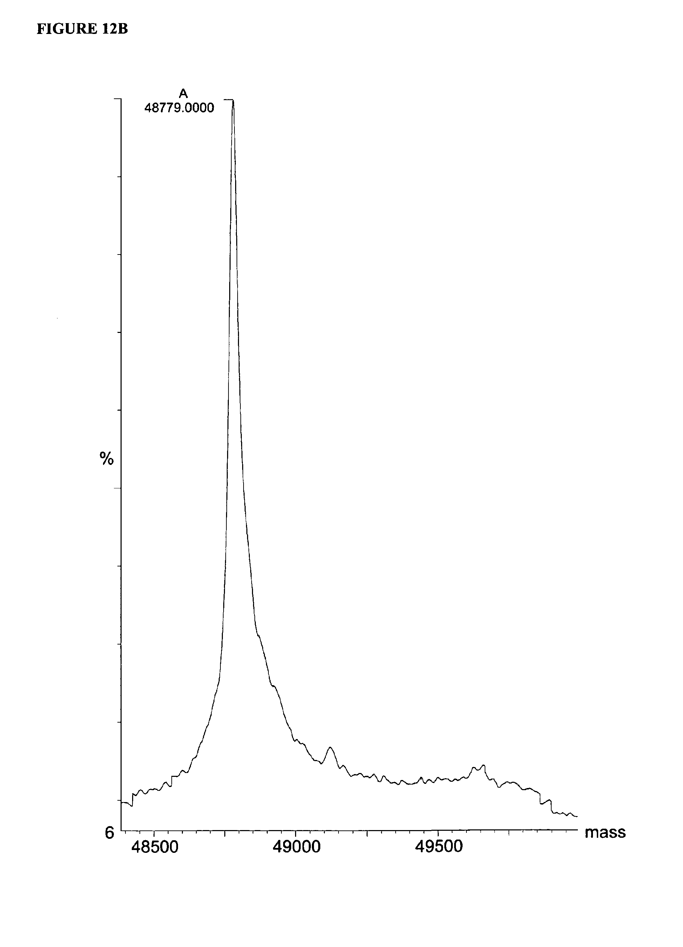

In Figures 12A and 12B, the heavy chain of Hz6F4-1(A) and Hz6F4-2(B) are digested by PNGase F to remove N-glycans. In both cases, the resulting peaks measured by mass spectrometry exhibits the expected masses for the respective polypeptide sequences. The remaining secondary peaks are in agreement with glycation site with an average of one or two hexoses. Again Hz6F4-2 (Figure 12B) shows a much higher homogeneity than Hz6F4-1 (Figure 12A).

### Example 11: In vivo activity of Hz6F4-2 on A-431 xenograft tumor model

A-431 epidermoid carcinoma cells were obtained from the American Type Culture Collection and were routinely cultured in DMEM (Lonza) supplemented with 10% heat inactivated Fetal Calf Serum (Sigma). Cells were maintained at 37°C in a humidified atmosphere of 95% air/5% CO2.

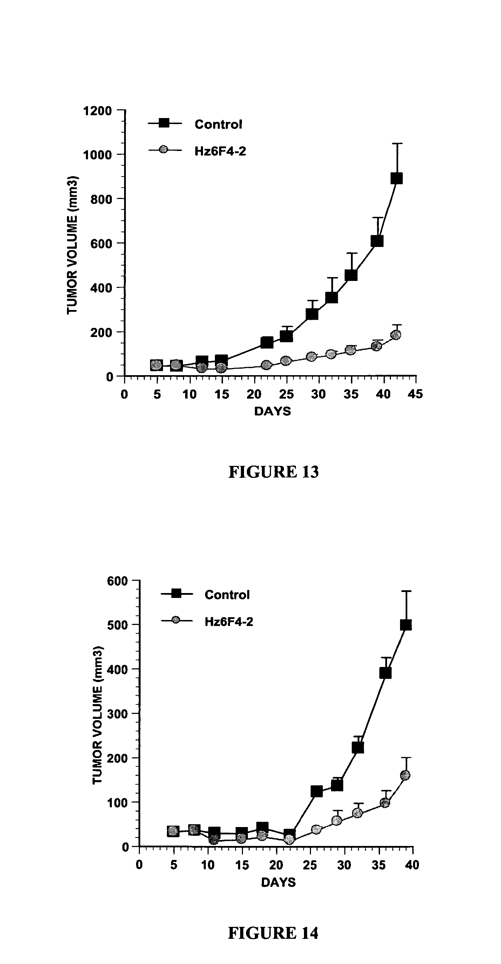

Athymic (Hsd:Athymic *Nude-Foxn1nu*) female nude mice (7 weeks old), obtained from Harlan Sprague Dawley, were housed in a light/dark cycle of 12/12 h and fed with sterilized rodent diet and water *ad libitum.* All procedures involving animals and their care were approved by the Institutional Animal Care and Use Committee of the Centre d'Immunologie Pierre Fabre. Mice were subcutaneously injected with 10 million A-431 cells in the exponential phase of growth, on the right flank of each mouse. After five days, healthy animals with similar tumor volumes were selected carefully and distributed into homogeneous tumor size groups of 6 animals each. Animals were treated i.p. with a loading dose of 2 mg/dose of Hz6F4-2 at D5 and 1 mg/dose per mice, twice/week until the end of the experiment. Control group was treated i.p. with the corresponding volume of PBS. Tumor volume was assessed twice a week using the formula: π/6.length.width.height. Results are represented in Figure 13.

### Example 12: In vivo activity of Hz6F4-2 on DU145 xenograft tumor model

DU145 prostate carcinoma cells were obtained from the American Type Culture Collection and were routinely cultured in DMEM (Lonza) supplemented with 10% heat inactivated Fetal Calf Serum (Sigma). Cells were maintained at 37°C in a humidified atmosphere of 95% air/5% CO2.

Swiss-Nude (Crl:NU(Ico)-*Foxnlnu*) female mice (8 weeks old), obtained from Charles River Laboratories France, were housed in a light/dark cycle of 12/12 h and fed with sterilized rodent diet and water *ad libitum.* All procedures involving animals and their care were approved by the Institutional Animal Care and Use Committee of the Centre d'Immunologie Pierre Fabre. Mice were subcutaneously injected with 10 million DU-145 cells in the exponential phase of growth, on the right flank of each mouse. After five days, healthy animals with similar tumor volumes were selected carefully and distributed into homogeneous tumor size groups of 6 animals each. Animals were treated i.p. with a loading dose of 2 mg/dose of Hz6F4-2 at D5 and 1 mg/dose per mice, twice/week until the end of the experiment. Control group was treated i.p. with the corresponding volume of PBS. Tumor volume was assessed twice a week using the formula : π/6.length.width.height. Results are represented in Figure 14.

## Claims

1. Method for the preparation of a high-homogeneous variant of a parental humanized antibody, or a derivative or antigen binding fragment thereof, said parental humanized antibody comprising a light chain variable domain having the 3 CDRs sequences SEQ ID Nos. 1, 2 and 3 and a heavy chain variable domain having the 3 CDRs sequences SEQ ID Nos.4, 5 and 6, and said humanized antibody being capable of binding to JAM-A, wherein said method comprises the step of:
a) the substitution with a neutral or basic amino acid residue of at least one amino acid residue of said parental humanized antibody light chain variable domain or of said parental humanized antibody heavy chain variable; and
b) the selection of the variant of said humanized antibody obtained in step a) as a high-homogeneous variant thereof whether said substitution results in the increase of the pI of the variant humanized antibody compared with the parental humanized antibody.

2. Method for the preparation of a high-homogeneous variant of a parental humanized antibody according to claim 1, wherein in step a):
- at least one amino acid residue selected from D1, Q3, Q24 and E55 of the light chain variable domain having the sequence SEQ ID No. 9 is substituted with a neutral or basic amino acid residue, and
- at least one amino acid residue selected from E1, Q38, G44, E62 and D77 of the heavy chain variable domain having the sequence SEQ ID No. 10 is substituted with a neutral or basic amino acid residue.

3. Method for the preparation of a high-homogeneous variant of a parental humanized antibody according to one of claims 1 to 2, wherein in step a):
- at least one amino acid residue selected from D1, Q3, Q24 and E55 of the light chain variable domain having the sequence SEQ ID No. 9 is substituted with the amino acid residue A, R, K and Q, respectively; and
- at least one amino acid residue selected from E1, Q38, G44, E62 and D77 of the heavy chain variable domain having the sequence SEQ ID No. 10) is substituted with the amino acid residue Q, R, R, Q and S, respectively.

4. The variant humanized antibody according to claim 3, or a derivative or antigen binding fragment thereof, **characterized in that** it comprises:
i) a light chain variable domain comprising the sequence SEQ ID N° 11, and
ii) a heavy chain variable domain comprising the sequence SEQ ID N° 12.

5. A variant humanized antibody according to claim 4, or a derivative or antigen binding fragment thereof, **characterized in that** its pI is comprised between 7 and 9, preferably between 7 and 8.

6. A variant humanized antibody according to one of claims 4 and 5, or a derivative or antigen binding fragment thereof, **characterized in that** it has a Kd for the JAM-A protein between roughly 100 nM and 100 pM, more preferentially between 50 nM and 1 nM.

7. An isolated nucleic acid **characterized in that** it is selected among the following nucleic acids:
a) a nucleic acid, DNA or RNA, coding for an antibody, or for a derivative or antigen binding fragment thereof, according to one of the claims 4 to 6;
b) a nucleic acid comprising the light chain of nucleic acid sequence SEQ ID No. 13 and the heavy chain of nucleic acid sequence SEQ ID No. 14;
c) a nucleic acid complementary to a nucleic acid as defined in a) or b).

8. A variant humanized antibody, or a derivative or antigen binding fragment thereof, according to one of the claims 4 to 7, for use as a drug.

9. A composition comprising as an active ingredient a compound consisting of a variant humanized antibody, or a derivative or antigen binding fragment thereof, according to one of the claims 4 to 8.

10. A composition according to claim 9, for use as a drug.

11. The use of a variant humanized antibody or a derivative or antigen binding fragment thereof, according to one of the claims 4 to 8, and/or of a composition according to any of the claims 9 or 10, for the preparation of a drug for the prevention or the treatment of a disease related to tumor cell proliferation.

12. The use according to claim 11, for the preparation of a drug for cancer prevention or treatment.

13. The use according to claim 12, **characterized in that** said cancer is a cancer selected among prostate cancer, osteosarcoma, lung cancer, breast cancer, endometrial cancer, multiple myeloma, ovarian cancer, pancreatic cancer and colon cancer.


SEQUENCE LISTING

<110>  Pierre Fabre Medicament

<120>  NOVEL HOMOGENEOUS HUMANIZED ANTIPROLIFERATION ANTIBODIES

<130>  68968/D29193

<160>  14

<170>  PatentIn version 3.3

<210>  1
<211>  6
<212>  PRT
<213>  mus musculus

<400>  1

Gln Asp Ile Asn Asn Tyr
1               5


<210>  2
<211>  3
<212>  PRT
<213>  mus musculus

<400>  2

Tyr Thr Ser
1


<210>  3
<211>  8
<212>  PRT
<213>  mus musculus

<400>  3

Leu Gln Tyr Asp Asn Leu Trp Thr
1               5


<210>  4
<211>  8
<212>  PRT
<213>  mus musculus

<400>  4

Gly Tyr Ser Phe Thr Asp Tyr Ser
1               5


<210>  5
<211>  8
<212>  PRT
<213>  mus musculus

<400>  5

Ile Asp Pro Tyr Asn Gly Gly Thr
1               5


<210>  6
<211>  9
<212>  PRT
<213>  mus musculus

<400>  6

Ala Arg Gln Thr Asp Tyr Phe Asp Tyr
1               5


<210>  7
<211>  106
<212>  PRT
<213>  mus musculus

<400>  7

Asp Ile Gln Met Thr Gln Ser Pro Ser Ser Leu Ser Ala Ser Leu Gly
1               5                   10                  15


Gly Lys Val Thr Ile Thr Cys Lys Ala Ser Gln Asp Ile Asn Asn Tyr
            20                  25                  30


Ile Ala Trp Tyr Gln His Lys Pro Gly Lys Gly Pro Arg Leu Leu Ile
        35                  40                  45


His Tyr Thr Ser Thr Leu Gln Ala Gly Ile Pro Ser Arg Phe Ser Gly
    50                  55                  60


Ser Gly Ser Gly Arg Asp Tyr Ser Phe Ser Ile Ser Asn Leu Glu Pro
65                  70                  75                  80


Glu Asp Ile Gly Thr Tyr Tyr Cys Leu Gln Tyr Asp Asn Leu Trp Thr
                85                  90                  95


Phe Gly Gly Gly Thr Lys Leu Glu Ile Lys
            100                 105


<210>  8
<211>  116
<212>  PRT
<213>  mus musculus

<400>  8

Glu Ile Gln Leu Gln Gln Ser Gly Pro Glu Leu Val Lys Pro Gly Ala
1               5                   10                  15

Ser Val Lys Val Ser Cys Lys Ala Ser Gly Tyr Ser Phe Thr Asp Tyr
            20                  25                  30

Ser Met Tyr Trp Val Lys Gln Ser His Gly Lys Ser Leu Glu Trp Ile
        35                  40                  45

Gly Tyr Ile Asp Pro Tyr Asn Gly Gly Thr Arg Tyr Asn Gln Lys Phe
    50                  55                  60

Lys Gly Lys Ala Thr Leu Thr Val Asp Lys Ser Ser Ser Thr Ala Phe
65                  70                  75                  80

Met His Leu Asn Ser Leu Thr Ser Glu Asp Ser Ala Val Tyr Tyr Cys
                85                  90                  95

Ala Arg Gln Thr Asp Tyr Phe Asp Tyr Trp Gly Gln Gly Thr Thr Leu
            100                 105                 110

Thr Val Ser Ser
        115

<210>  9
<211>  106
<212>  PRT
<213>  artificial sequence

<220>
<223>  humanized light chain

<400>  9

Asp Ile Gln Met Thr Gln Ser Pro Phe Ser Leu Ser Ala Ser Val Gly
1               5                   10                  15

Asp Arg Val Thr Ile Thr Cys Gln Ala Ser Gln Asp Ile Asn Asn Tyr
            20                  25                  30

Ile Ala Trp Tyr Gln Gln Lys Pro Ala Lys Ala Pro Lys Leu Phe Ile
        35                  40                  45

His Tyr Thr Ser Thr Leu Glu Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Leu Gln Tyr Asp Asn Leu Trp Thr

85                  90                  95

Phe Gly Gln Gly Thr Lys Val Glu Ile Lys
            100                 105

<210>  10
<211>  116
<212>  PRT
<213>  artificial sequence

<220>
<223>  humanized heavy chain

<400>  10

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Asp Tyr
            20                  25                  30

Ser Met His Trp Val Gln Gln Ala Pro Gly Gln Gly Leu Glu Trp Met
        35                  40                  45

Gly Tyr Ile Asp Pro Tyr Asn Gly Gly Thr Arg Tyr Ser Glu Lys Phe
    50                  55                  60

Gln Gly Arg Val Thr Ile Thr Ala Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Asp Tyr Phe Asp Tyr Trp Gly Gln Gly Thr Leu Val
            100                 105                 110

Thr Val Ser Ser
        115

<210>  11
<211>  106
<212>  PRT
<213>  artificial sequence

<220>
<223>  humanized light chain

<400>  11

Ala Ile Arg Met Thr Gln Ser Pro Phe Ser Leu Ser Ala Ser Val Gly
1               5                   10                  15

Asp Arg Val Thr Ile Thr Cys Lys Ala Ser Gln Asp Ile Asn Asn Tyr
            20                  25                  30

Ile Ala Trp Tyr Gln Gln Lys Pro Ala Lys Ala Pro Lys Leu Phe Ile
        35                  40                  45

His Tyr Thr Ser Thr Leu Gln Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Leu Gln Tyr Asp Asn Leu Trp Thr
                85                  90                  95

Phe Gly Gln Gly Thr Lys Val Glu Ile Lys
            100                 105

<210>  12
<211>  116
<212>  PRT
<213>  artificial sequence

<220>
<223>  humanized heavy chain

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Asp Tyr
            20                  25                  30

Ser Met His Trp Val Arg Gln Ala Pro Gly Gln Arg Leu Glu Trp Met
        35                  40                  45

Gly Tyr Ile Asp Pro Tyr Asn Gly Gly Thr Arg Tyr Ser Gln Lys Phe
    50                  55                  60

Gln Gly Arg Val Thr Ile Thr Ala Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Asp Tyr Phe Asp Tyr Trp Gly Gln Gly Thr Leu Val

            100                 105                 110

Thr Val Ser Ser
        115

<210> 13
<211> 318
<212> DNA
<213> artificial sequence

<220>
<223> humanized light chain

<400> 13
gccattagga tgactcagtc ccccttcagc ctcagcgcct cagtcggtga cagagtgact 60

ataacatgta aggcttcaca agacatcaac aactatattg catggtacca gcaaaagcca 120

gccaaagctc ccaaactttt tatccattac acctccacac tgcagagtgg cgttccttcc 180

cggtttagtg gatctggttc tggcaccgat tacaccctga ccatcagctc tctgcagcct 240

gaagatttcg ccacatatta ttgccttcag tacgataatt tgtggacttt cggccagggg 300

accaaggtgg agatcaag 318

<210> 14
<211> 348
<212> DNA
<213> artificial sequence

<220>
<223> humanized heavy chain

<400> 14
caggtgcaat tggtacagtc aggcgcggag gtgaagaagc ctggggctag tgttaaagtc 60

tcctgtaaag cctccggata ttccttcact gactactcta tgcattgggt tcgccaggca 120

ccagggcagc ggctggaatg gatggggtac attgatccct acaacggagg cacgcgatat 180

agtcagaagt tccagggtcg ggtgacaatc acagccgata cgtccaccag caccgcctac 240

atggagttga gcagtctcag gtcagaagac acagccgtgt actattgcgc aagacagacc 300

gattatttcg actactgggg ccaaggcact ctcgtgaccg tctctagc 348